(19) Europäisches Patentamt
European Patent Office
Office européen des brevets

(11) **EP 3 481 352 B1**

(12) **EUROPEAN PATENT SPECIFICATION**

(45) Date of publication and mention
of the grant of the patent:
**15.09.2021 Bulletin 2021/37**

(21) Application number: **16908273.2**

(22) Date of filing: **08.07.2016**

(51) Int Cl.:
*A61F 13/51* (2006.01)          *A61F 13/84* (2006.01)
*A61L 15/34* (2006.01)          *A61L 15/42* (2006.01)
*A61F 13/511* (2006.01)          *A61L 15/22* (2006.01)

(86) International application number:
**PCT/SE2016/050706**

(87) International publication number:
**WO 2018/009112 (11.01.2018 Gazette 2018/02)**

(54) **HYGIENE ARTICLE COMPRISING SOLID EMULSION AND METHOD OF MANUFACTURE**

HYGIENEARTIKEL MIT FESTER EMULSION UND VERFAHREN ZUR HERSTELLUNG

ARTICLE D'HYGIÈNE COMPRENANT UNE ÉMULSION SOLIDE ET PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**15.05.2019 Bulletin 2019/20**

(73) Proprietor: **ESSITY HYGIENE AND HEALTH AKTIEBOLAG**
**405 03 Göteborg (SE)**

(72) Inventors:
• **ABBAS, Shabira**
**405 03 Göteborg (SE)**
• **HAGBERG, Daniel**
**405 03 Göteborg (SE)**
• **HJELM JONASSON, Simon**
**412 79 Göteborg (SE)**
• **BORDES, Romain**
**416 48 Göteborg (SE)**

(74) Representative: **Nederlandsch Octrooibureau**
**P.O. Box 29720**
**2502 LS The Hague (NL)**

(56) References cited:
**US-A1- 2004 266 302      US-A1- 2004 266 302**
**US-A1- 2010 221 309      US-A1- 2011 236 562**
**US-A1- 2015 273 420      US-B1- 6 375 963**

• **ZHEN HU ET AL: "Synergistic Stabilization of Emulsions and Emulsion Gels with Water-Soluble Polymers and Cellulose Nanocrystals", ACS SUSTAINABLE CHEMISTRY & ENGINEERING, vol. 3, no. 5, 4 May 2015 (2015-05-04), pages 1023-1031, XP055385774, US ISSN: 2168-0485, DOI: 10.1021/acssuschemeng.5b00194**
• **ZHEN HU ET AL.: 'Synergetic Stabilization of Emulsions and Emulsion Gels with Water soluble Polymers and Cellulose Nanocrystals' ACS SUSTAINABLE CHEMISTRY AND ENGINEERING vol. 3, no. 5, 04 May 2015, pages 1023 - 1031, XP055385774**

EP 3 481 352 B1

**Description**

TECHNICAL FIELD

The present disclosure relates to hygiene articles comprising at least one layer.

BACKGROUND ART

In the field of hygiene articles of the type comprising at least one layer, it is often desired to provide active agents, which can improve the function of the article or enhance the user's experience when using the article. Active agents in the form of e.g. skin well-being agents, odour control agents, or perfumes can be applied to hygiene articles, such as feminine hygiene articles, baby diapers, incontinence guards, or wipes, for example by spraying onto the surface of the article, or by impregnating one or more layers with the active agent.

US 2004/266302 describes a disposable absorbent article containing an encapsulated essential oil where upon mechanical breaking of the encapsulation or upon exposure to liquid, fragrance and related antimicrobial properties contained therein are released. Also, Hu et al. (Synergetic Stabilization of Emulsions and Emulsion Gels with Water Soluble-Polymers and Cellulose Nanocrystals, ACS Sustainable Chemistry and Engineering, vol. 3, no. 5, 4 May 2015, pages 1023-1031) describes stable emulsions comprising nanocellulose and cellulose derivatives in a ratio of 3:2, which is shown to lead to smaller oil droplets and enhanced emulsion stability compared to polymers alone.

SUMMARY OF THE INVENTION

The present invention relates to a hygiene article according to the features of claim 1. The nanocellulose is preferably cellulose nanocrystals (CNC). The cellulose derivatives (CD) are preferably chosen from carboxymethyl cellulose (CMC), hydroxyethyl cellulose (HEC), ethyl hydroxyethyl cellulose (EHEC), or hydroxylpropyl methyl cellulose (HPMC), preferably CMC or HEC, or combinations of two or more of these cellulose derivatives.

The solid continuous phase of the solid emulsion comprises 0,5-50 wt% nanocellulose, which may be nanocrystals (CNC), more preferably 3-40 wt%, most preferably 10-30 wt%, based on the weight of cellulose derivatives (CD) comprised in the solid continuous phase, and 50-99,5 wt% cellulose derivatives (CD) based on the total dry content weight of the solid continuous phase. The weight ratio of the dispersed oil phase to the dry content in the continuous phase of the solid emulsion is advantageously 20:80-1:99 wt/wt. Release of the dispersed oil phase is preferably initiated, when the a pressure above a critical pressure value is applied to the solid emulsion comprised in the article, wherein the critical pressure value preferably is 0,1 kPa.

The oil containing composition of the liquid dispersed phase preferably comprises one or more active agents chosen from but not limited to: skin care agents, odour control agents, antibacterial agents, wettability agents, detection agents, indication agents, lubrication agents, agents contributing to mechanical properties of the absorbent product, enzyme inhibiting agents, pH controlling agents.

In the hygiene article, the at least one layer comprised in the article is preferably a fibrous layer or a foam layer. The solid emulsion may be present in article adhered to fibres in a fibrous layer, or may be in the form of a film, or a foam, or in the form of particles.

The hygiene article may comprise two or more layers, wherein at least one layer comprises the solid emulsion, and wherein the layer comprising the solid emulsion is a fibrous layer. The fibrous layer may be a nonwoven material, preferably comprised of hydrophobic fibres, or it may be a tissue layer.

The hygiene article may be an absorbent hygiene article, _comprising a body facing layer and an absorbent core, in which the solid emulsion is present in the body facing layer. The solid emulsion may then be present in an area of the body facing layer covering the absorbent core, or an area of the body facing layer not covering the absorbent core, such as in standing gathers or wings. The solid emulsion may be present in the absorbent article in an amount of 0,01-20 g/m$^2$ of the body facing layer.

The hygiene article may alternatively be a wipe or tissue product comprising one or more layers. The solid emulsion is then preferably present in an amount of 0,01-20 g/m$^2$ of a layer in the product. A wipe or tissue product may further comprise one or more intermediate layers, arranged between first and second outer layers, and wherein the solid emulsion is present in one or more of the intermediate layers.

The present invention also relates to a method of manufacture of a fibrous layer for use as a component in the hygiene article, described above, according to method claim 13.

The present invention also relates to a method of manufacture of the hygiene article, described above, according to method claim 14. The method may further comprise applying the fluid emulsion by printing, roll coating or spraying.

The present invention also relates to an alternative method of manufacture of the hygiene article, describe above, according to method claim 15.

In the methods described above the continuous phase of the fluid emulsion preferably comprises 0,1-0,8 wt% cellulose nanocrystals (CNC) and 1-3 wt% cellulose derivatives (CD), based on the total weight of the fluid emulsion, and the weight ratio of the dispersed oil phase to the emulsion is preferably 5-50 wt/wt.

BRIEF DESCRIPTION OF THE DRAWINGS

Fig. 1 schematically illustrates cellulose derivatized into hydroxyethyl cellulose;

Fig. 2 is a schematic illustration of the process of making a solid emulsion through solvent casting of the continuous phase;

Fig. 3 illustrates the magnitude of the initial backscattered light of emulsions with given composition of CDs and CNCs with a dodecane content of 25% wt;

Fig. 4 illustrates the deduced character of emulsions droplets with different CDs;

Fig. 5 shows the compressive stress vs strain relation of solid emulsions made with 0.5% CNCs and four different CDs (2.0%);

Fig. 6 shows the relation between compressive strain and relative mass loss due to compression in solid emulsions made with 2.0% of given CD and 0.5% CNCs;

Fig. 7 shows stress-strain curves for tested emulsion;

Fig. 8 shows mass loss as a function of compressive strain for tested emulsion;

Fig. 9 shows the compressive stress vs strain relation for the same type of solid emulsion (2.0% CMC 0.5% CNCs;

Fig. 10 shows CLSM micrographs of solid emulsions made with 2.5% CMC and 2.0% CMC + 0.5% CNC;

Fig. 11 shows CLSM micrographs of solid emulsions made with 2.0% HEC + 0.5% CNC and 2.0% EHEC + 0.5% CNC;

Fig. 12 shows the cell diameter distribution for solid emulsions with EHEC/CNC and HEC/CNC;

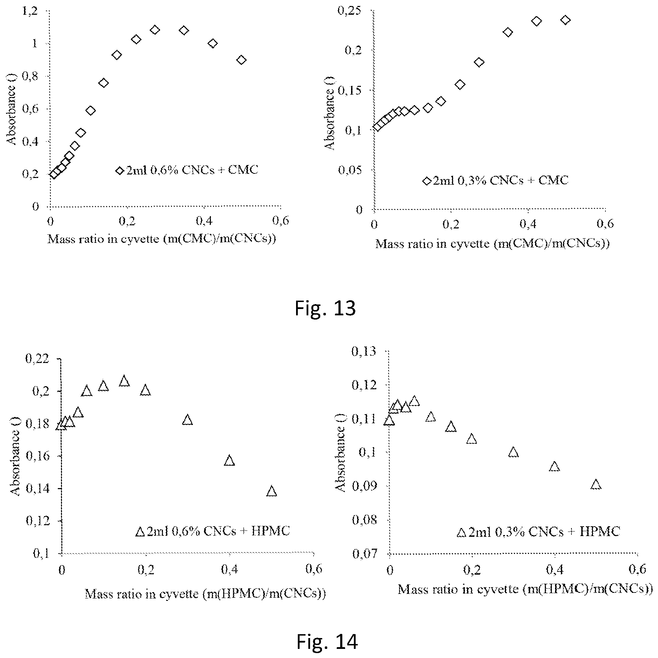

Fig. 13 shows the absorbance of CNC-CMC mixtures as a function of amount of CMC, expressed as mass fraction CMC;

Fig. 14 shows the absorbance of CNC-HPMC mixtures at 400 nm as a function of amount of HPMC, expressed as mass fraction CMC;

Fig. 15 is an illustration of the two possible characteristics of the solid contents in solid emulsions;

Fig. 16 is an illustration of hypothetical behavior of solid emulsions with varying CNC/CD-ratio;

Fig. 17a is an ESEM image of a solid emulsion specimen;

Fig. 17b is an enlargement of the center portion of Fig. X.1a;

Fig. 17c is an ESEM image of the same specimen as in Fig. 17a after compression;

Fig. 18 is an ESEM image of a foamed emulsion specimen;

Fig. 19a-c are ESEM images of a PP nonwoven treated with emulsion;

Fig. 20 is an ESEM image of a PET nonwoven treated with emulsion;

Fig. 21a is an ESEM image of a PUR foam treated with emulsion;

Fig. 21b is an enlargement of a portion of Fig. 21a;.

Fig. 22 is an ESEM image of a tissue treated with emulsion;

Fig. 23a-b are ESEM images of a tissue nonwoven treated with emulsion;

Fig. 24 in an ESEM image of a PP nonwoven treated with excess of emulsion.

DETAILED DESCRIPTION

It has been found that it is possible to encapsulate liquid oil in solid cellulose derivative polymer matrices by producing oil-in-water emulsions stabilized by cellulose nanocrystals with the water soluble derivative in the continuous phase. The polymer will then form a solid matrix upon the drying of the emulsion, in which the oil remains encapsulated. The resulting system is referred to as a solid emulsion or dry emulsion, and the general technology associated with the production of this and other types of emulsion-originated materials is called emulsion-templating. Materials produced via this method are inherently influenced by the characteristics of the original emulsion which can be varied by many means, such as choice of dispersed and continuous phase, type of emulsifier and stabilizer and also processing and preparation method of the original emulsion.

The present disclosure relates to hygiene articles comprising a certain solid emulsion as described in more detail below, and to methods of manufacturing such articles. The solid emulsion comprises a matrix of solid continuous phase and a liquid dispersed oil phase, wherein the solid continuous phase comprises cellulose derivatives (CD) and nanocellulose, which is preferably cellulose nanocrystals (CNC), and the liquid dispersed oil phase is an oil containing composition.

Generally, a suitable emulsion is formed according to the current disclosure by firstly dissolving at least one polymeric cellulose derivative (referred to herein as "cellulose derivative", such as HPMC) in water. It will be understood that by "water" it is meant a substantially aqueous system where very small amounts of impurities (for example other water miscible solvents) may be present.

In some alternative aspects, alternative water-soluble polymers may be used instead of a polymeric cellulose derivative. Suitable alternative water soluble polymers include synthetic polymers as well as those derived from natural materials. One example of a suitable alternative water soluble polymer is polyvinyl alcohol, PVA. Suitable properties of such alternative polymers may be those described below for cellulose derivatives.

Nanocellulose can be microfibrillated cellulose (MFC) or cellulose nanocrystals (CNC). The cellulose derivatives (CD) are preferably chosen from carboxymethyl cellulose (CMC), hydroxyethyl cellulose (HEC), ethyl hydroxyethyl cellulose (EHEC), or hydroxylpropyl methyl cellulose (HPMC), preferably CMC or HEC, or combinations of two or more of these cellulose derivatives. The solid continuous phase of the solid emulsion comprises 0,5-50 wt% nanocellulose, which is preferably nanocrystals (CNC), preferably 3-40 wt%, and most preferably 10-30 wt%, based on the weight of cellulose derivatives (CD) comprised in the solid continuous phase. The solid emulsion preferably comprises 50-99,5 wt% cellulose derivatives (CD) based on the total dry content weight of the solid continuous phase. The weight ratio of the dispersed oil phase to the dry content in the continuous phase of the solid emulsion is preferably 20-99 wt/wt.

The oil containing composition of the liquid dispersed phase preferably comprises one or more active agents, such as, but not limited to, skin care agents, odour control agents, antibacterial agents, wettability agents, detection agents, indication agents, lubrication agents, agents contributing to mechanical properties of the absorbent product, enzyme inhibiting agents, pH controlling agents.

Skin care agents comprised in the emulsion, preferably the oil containing phase can include lipids (including but not limited to fats, oils, waxes), solvents (including but not limited to water), water-soluble substances, surface-active agents (including but not limited to emulsifiers, surfactants), viscosity-regulating substances, pH-regulating substances, preserving agents, complexing agents (e. g. chelate), delivery systems (e. g. liposomes, microcapsules, etc.), pigments, perfumes, and active substances (including pharmaceutical agents).

The lipids are usually emulsified in water, known as oil/water emulsion, or water is emulsified in the lipid phase, known as water/oil emulsion.

Lipid skin care agents can be paraffins (alkanes) with 12-35 carbons, such as, but not limited to, paraffin oil (mineral oil) or petrolatum (e.g., VASELINE®).

Other examples of suitable lipid skin care agents are triglycerides, which may be refined and/or hydrogenated, of animal or vegetable origin, preferably with carbon chain lengths C-18 or less, such as, but not limited to milk fat, coconut oil (Cocous nocifera), palm-kernel oil (Elaeis guineeis), or caprylic triglycerid; animal or vegetable lipids with unsaturated C-18 fatty acids, such as, but not limited to, Japan wax (Rhus succesdanes), tallow fat, soybean oil (Glycerin soya), peanut oil (Arachais hypogaea), maize oil (Zea mays), sunflower oil (Helanthus annus), grapeseed oil (Vitis vinifera), safflower oil (Carthamus tinctorius), sweet almond oil (Prunnus amygdalus dulcis), hazelnut oil (Corylus americana), walnut oil (Juglans regia), olive oil (Olea europasa), avocado oil (Persea gratissima), sesame oil (Sesamum indicum), cottonseed oil (Gopssypium), palm oil (Elaesis guineensis), rice oil (Oryza sativa), rape oil (Canola), apricot-kernel oil (Prunus armeniaca), cocoa butter (Theobroma cao), shea butter (Butyrospermum parkii), wheatseed oil (Triticum vulgare), or Bassia latifola oil; or animal or vegetable lipids with carbon chains over C-18, such as, but not limited to, beeswax Cera alba), seed oil (Limnanthes alba), rapeseed oil (Brassica capmestris), cucumberseed oil (Borago officinalis), linseed oil (Linum usitatissimum), ricin oil (Ricinus communis), veronia oil (Veronia galamensis), jojoba oil (Buxus chinensis), candlewax (Euphorbia cera), or ongokea oil (Ongokea gore).

The skin care agents may also be fatty alcohols with straight or branched carbon chain lengths of 12-32 carbons, for example, cetyl alcohol, stearyl alcohol or a mixture thereof, or fatty acid esters with 12-32 carbons, for example, methyl palmitate, methyl stearate, isopropyl myristate, isopropyl laurate, isopropyl palmitate, isopropyl stearate, octyl palmitate, octyl stearate or octyl laurate.

The skin care agents may also be polyalcohols, for example sugar alcohols or polyglycerols.

The skin care agents may also be complex lipids, for example, phospholipids or sphingolipids (ceramides); or waxes, for example of animal origin, for example beeswax or lanolin, or of vegetable origin, for example carnauba or candelilla, or of mineral origin, for example ozocerite or ceresin.

The skin care agents may also be polysiloxanes, which may be straight, branched or cyclic. Examples are polydimethyl-siloxane (dimethicone) or polydiethylsiloxane.

Skin care agents can include emulsions, such as, but not limited to, emulsions of one or more fat, with hydrophilic substances, such as, but not limited to, water, glycerol, polyethylene glycol (PEG), propylene glycol, butylene glycol, sorbitol, silicone glycols, or the like, or mixtures thereof.

The skin care agents can include pH-regulating additives, for example organic or inorganic acids, such as, but not limited to, adipic acid, ascorbic acid, benzoic acid, citric acid, malic acid, tartaric acid, lactic acid, phosphoric acid, or hydrochloric acid; or buffers, made for example from said acids with their corresponding salts. The skin care agents can also include polymeric acids, for example polyphosphoric acid or polyacrylic acid. The skin care agents can also include alkaline substances such as ammonium hydroxide or calcium carbonate.

Skin care agents can also include additions of probiotic microorganisms, characterized by being antagonistic towards undesired microorganisms, e. g. skin infection pathogens. Examples of probiotic microorganisms which can be used are individual strains or mixtures of several strains of lactic acid bacteria taken from the species Lactobacillus acidophilus, Lactobacillus curvatus, Lactobacillus plantarum or Lactococis lactis.

Skin care agents can also include active substances, such as, but not limited to, antiinflammatory agents, e. g. acetylsalicylic acid, allantoin, azulen, alpha- bisabolol (chamomile), flavonoids, glycyrrhizinic acid, ichthammol (Inotyol)), tannins, or astringents (vasoconstrictors), for example TiO, ZnO (and other Zn compounds), aluminum acetate solution, aluminum tartrate solution (and other Al compounds), ethanol or ethanol-based solutions.

Skin care agents can also include nourishing agents such as, but not limited to, Aloe vera (Aloe barbadensis), alpha-hydroxy acids, for example citric acid, tartaric acid, lactic acid, malic acid, etc.; or algae extract, ascorbic acid (vitamin C), or vitamin A compounds, for example retinol, retinal, tretinoin and isotretinoin, or avocado sterols, betaine (trimethylglycine), ceramides, grapeseed extract, essential fatty acids, flavonoids, phytosphingosine, phytosterols, hyaluronic acid, yeast extract, chitosan, milk protein (Lactis proteinum), pantenol (provitamin B5), polysaccharides, rosemary extract, tocopherol (vitamin E), ubiquinone (coenzyme Q10), urea.

Skin care agents can also include calendula officinalis flower extract, mannitol, ammonium glycyrrhizate, caffeine, zinc gluconate, aesculus hippocastanum extract, tocopheryl acetate.

Skin care agents can also consist of ready-made mixtures of skin ointments, creams and lotions, for example, TENA Skin Cream, which include the ingredients: Aqua, Canola Oil, Glycerin, Ethylhexyl Stearate, Glyceryl Stearates, Zea Mays Oil,Polyglyceryl-3 Methylglucose Distearate, Cetearyl Alcohol, Dicaprylyl Carbonate, Hydrogenated Coco-Glycerides, Panthenol, Tocopheryl Acetate, Hydroxyethyl Acrylate/Sodium Acryloyldimethyl Taurate Copolymer, Betaine, Parfum, Xanthan Gum, Sodium Citrate, Phenoxyethanol, Benzoic Acid, Dehydroacetic Acid, Tocopherol, Citric Acid, Sorbitan Isostearate, Polysorbate 60, Pantolactone; or TENA Skin Cream Perfume free which include the ingredients; Aqua, Canola Oil, Glycerin, Ethylhexyl Stearate, Glyceryl Stearates, Zea Mays Oil,Polyglyceryl-3 Methylglucose Distearate, Cetearyl Alcohol, Dicaprylyl Carbonate, Hydrogenated Coco-Glycerides, Panthenol, Tocopheryl Acetate, Hydroxyethyl Acrylate/Sodium Acryloyldimethyl Taurate Copolymer, Betaine, Xanthan Gum, Sodium Citrate, Phenoxyethanol, Benzoic Acid, Dehydroacetic Acid, Tocopherol, Citric Acid, Sorbitan Isostearate, Polysorbate 60, Pantolactone; or TENA Skin Lotion, which include the ingredients: Aqua, Dicaprylyl Carbonate, Polyglyceryl-3 Methylglucose Distearate, Ethylhexyl Stearate, Glycerin, Glyceryl Stearates, Canola Oil, Cetearyl Alcohol, Tocopheryl Acetate, Hydroxyethyl Acrylate/Sodium Acryloyldimethyl Taurate Copolymer, Panthenol, Parfum, Xanthan Gum, Sodium Citrate, Phenoxyethanol, Benzoic Acid, Dehydroacetic Acid, Tetrasodium Iminodisuccinate, Tocopherol, Citric Acid, Sorbitan Isostearate, Polysorbate 60, Pantolactone. TENA products are sold commercially by SCA Hygiene Products, Gothenburg, Sweden.

It should be noted that this disclosure is not limited to the skincare agents mentioned above, and that instead these are just examples of skincare agents that could be used.

Odour control agents that can be of use within the scope of the present disclosure include bacteria growth inhibitors and/or moisture-insensitive odour inhibitors. Bacteria growth inhibitors can include, but are not limited to, one or more inhibitors selected from the group that includes: chlorhexidine, quaternary ammonium compounds, cupric salts, silver and silver salts, chelating agents, parabens, e.g. methyl, ethyl, or propyl parabens, chitin, and pH buffered materials.

Other active agents that can be comprised in the oil containing composition are antibacterial agents; such as Barberry, Blueberry, Burdock, Cinnamon, Chamomile, Cranbarry, Dill, Eucalyptus, Oak, Olive oil. Furthermore, other active agents are suitably wettability agents, such as nonionic, cationic, anionic and zwitterionic surfactants or surface active polymers; detection and indication agents, such as halochromic dyes, alizarin, Phenol Red, Bromothymol Blue or Cresol Red; agents contributing to mechanical properties of the absorbent product, lubricating agents such as fatty acids, dimethicone, triglyceride, fatty esters or fatty alcohols; enzyme inhibiting agents such as potato extract and zinksalizylate; pH controlling agents, organic acids such as lactic acid, citric acid, also inorganic acids or alkaline substances such as ammonium hydroxide or calcium carbonate.

The solid continuous phase of the solid emulsion can withstand compressive pressure to a certain extent. When the solid emulsion is subjected to compression above a critical pressure value the solid continuous phase ruptures gradually and the oil containing composition of the dispersed oil phase can leak out of the solid emulsion, and is thereby released. By incorporating a solid emulsion in the hygiene article, which releases the oil containing composition at a compressive pressure which corresponds to a compressive pressure which is applied to the solid solution during normal use of the hygiene article, an oil containing composition can be initially incorporated in the article, while the release of oil containing composition can be delayed until it is actually needed.

Release of the oil containing composition of the dispersed oil phase is initiated when a pressure above a critical pressure value is applied to the solid emulsion comprised in the article. The critical pressure value is preferably 0,1 kPa, more preferably within the range of 0,5kPa - 1 MPa. The critical pressure value for the solid emulsion can be adjusted according to the requirements for the type of hygiene article by selecting combinations of CDs as appropriate is dependent on product, structure and use of product and several other parameters. The pressure applied during use is normally approximately 2,5kPa-10kPa for baby diapers, 2,5kPa-100kPa for incontinence guards, and 2,5kPa-50kPa for wipes, such as tissue handkerchiefs.

As a complementary note it is realized that the critical pressure can also be reached by application of friction and/or shear forces, which is considered to be within the capacity of a man with ordinary skills in the art and not described further in detail here.

The hygiene article of the present disclosure may be an absorbent hygiene article or comprising one or more layers a wipe or tissue product comprising one or more layers. The hygiene article may also be a non-absorbent hygiene article comprising one or more layers configured to be worn in contact with the skin of the human or animal body.

The hygiene article comprises at least one layer, which may be a fibrous layer or a foam layer. The solid emulsion may be present in the article adhered to the fibers in a fibrous layer, or in the form of a film, or a foam, or in the form of particles. The hygiene article may also comprise two or more layers, wherein at least one layer comprises the solid emulsion. The layer comprising the solid emulsion may preferably be a fibrous layer, such as a nonwoven material, preferably comprised of hydrophobic fibers. The fibrous layer may alternatively be a tissue layer.

When the hygiene article is an absorbent hygiene article, it may comprise a body facing layer and an absorbent core, and the solid emulsion is then preferably present in the body facing layer, which does typically not store liquid during use, but it can also be located in another part of the article if desired. The solid emulsion can be present in an area of the body facing layer which covers the absorbent core, or in an area of the body facing layer not covering the absorbent core, such as in standing gathers or wings. The solid emulsion can be present in an amount of 0.01-20 g/m$^2$ of the body facing layer. Examples of absorbent hygiene articles, in which the solid emulsion can be incorporated, include but are not limited to incontinence guards, diapers, and feminine hygiene articles, such as sanitary napkins or panty liners, bed protectors and seat covers. The oil containing composition of the dispersed oil phase will typically be released when the article is subjected to pressure between the body of the user and a hard or stiff surface, for example when the user sits on a chair.

The hygiene article may comprise an absorbent composite material, such as the one described in WO2013009225A1. This absorbent composite material comprises an intimate mixture of network-forming cellulose fibrils (30-70 wt-%) having a diameter of greater than 100 $\mu$m, a hydrophilic absorbent polymer component (30-60 wt-%) bonded to the cellulose fibrils, a water soluble polymer component (30-60 wt-%). According to the present disclosure, active agents comprising odor inhibiting agents, anti-microbial agents, perfumes, skin care agents, odor reducing agents and nutrients for lactic acid producing bacteria can be contained in the oil containing composition, and are released when the solid emulsion is subjected to pressure. The absorbent composite material is useful as a combined liquid absorbing structure in an absorbent article such as a sanitary napkin or a panty liner and as an active agent release material.

Another example of an absorbent structure that can be used within the scope of the present disclosure is a superabsorbent polymer composite such as the one described in WO2010071584A1, a superabsorbent polymer composite comprising a superabsorbent polymer and cellulosic fibrils, where the cellulosic fibrils are nanofibrils having a diameter of less than or equal to 100 nm.

When the hygiene article is a wipe or tissue product comprising one or more layers, the solid emulsion may then be present in an amount of 0.01-20 $g/m^2$ of a layer in the product. The wipe or tissue product may further comprise one or more intermediate layers, arranged between first and second outer layers, and wherein the solid emulsion is present in one or more of the intermediate layers. Examples of wipe or tissue product, in which the solid emulsion can be incorporated, include but are not limited to dry wipes, such as facial wipes, dry baby wipes, cleaning wipes, dust cloths, or tissue products, such as kitchen paper, paper towels, or table napkins. The oil containing composition of the dispersed oil phase will typically be released when the article is squeezed by the hand of the user.

By including active agents in an oil containing composition entrapped within the solid continuous phase in the present solid emulsion, the physical properties of the material structures comprised in the hygiene article can be altered upon release of the active agent. For example, when the use of a hydrophobic skin care agent is desired in an absorbent hygiene article, the original hydrophilic properties of the absorbent structure can be retained, thus maintaining good absorbent properties, until the hydrophobic skin care agent is released. When the hygiene article is wipe or tissue product, the product can first be used as a dry wipe, and any original hydrophilic properties are maintained, until the user squeezes the wipe or tissue, so that the active agent(s) are released.

The solid continuous phase of the solid emulsion is hydrophilic, amphiphilic, predominately water soluble and liquid permeable. The oil containing composition of the dispersed phase is hydrophobic, and is preferably defined by a required HLB (hydrophilic-lipophilic balance) value of oil/water emulsion of 6-17. A hydrophilic substance has an affinity for water; readily absorbing or dissolving in water, and a hydrophobic substance lacks affinity for water; tending to repel and not absorb water; tending not to dissolve in or mix with or be wetted by water. The contact angle ($\theta$) is the angle at which the liquid-vapor interface meets the solidliquid interface. The contact angle is determined by the result between adhesive and cohesive forces. As the tendency of a drop to spread out over a flat, solid surface increases, the contact angle decreases. Thus, the contact angle provides an inverse measure of wettability. A contact angle less than 90° (low contact angle) usually indicates that wetting of the surface is very favorable, and the fluid will spread over a large area of the surface. Contact angles greater than 90° (high contact angle) generally means that wetting of the surface is unfavorable, so the fluid will minimize contact with the surface and form a compact liquid droplet. For water, a wettable surface may also be termed hydrophilic and a non-wettable surface hydrophobic. Table 1 describes varying contact angles and their corresponding solid/liquid and liquid/liquid interactions.

**Table 1**

| Contact angle | Degree of wetting | Strength of: Solid/liquid interactions | Strength of Liquid/liquid interactions |
|---|---|---|---|
| $\theta = 0$ | Perfect wetting | strong | weak |
| $0 < \theta < 90°$ | High wettability | strong | strong |
| | | weak | weak |
| $90° \leq \theta < 180°$ | low wettability | weak | strong |
| $\theta = 180°$ | perfectly non-wetting | weak | strong |

A material that is homogeneous gives a contact angle, whereas the same material in porous form gives a different contact angle. The contact angle of a fiber or fiber network (nonwoven web) can be measured. The hydrophilic-lipophilic balance of a surfactant is a measure of the degree to which it is hydrophilic or lipophilic, determined by calculating values for the different regions of the molecule. Methods for doing this are available in the art.

The versatility of CNCs functions to aid in the emulsification of the oil within the cellulose derivative polymer matrix, and to provide the matrix with mechanical strength and inherently enhanced encapsulation properties once it has solidified. The enhanced mechanical properties of the solid emulsions is partly influenced by the mechanical properties of the CNCs, which have been found to be within range of other commercial reinforcing agents, such as kevlar, carbon fibers, steel wires and boron nanowhiskers.

Cellulose is a linear homopolymer consisting of $\beta$-1,4-linked anhydroglucose as the repeating unit, where the individual anhydroglucose units are linked through an oxygen in a $\beta$-1,4 configuration. The amount of repeating units varies between 10000 and 15000, depending on the source of the cellulose. The relatively large amount of hydrogen and oxygen present in the polymer renders intermolecular aggregates stable in the sense that both intra- and intermolecular forces gets reinforced by extensive and complex hydrogen bonding. This chemistry also results in characteristic hierarchical structure of cellulose containing materials where cellulose chains packs in sheets and form highly crystalline assemblies known as elementary fibrils. These elementary fibrils further make up the structure of microfibrils which are characterized by multiple elementary fibrils connected through amorphous segments of cellulose.

Cellulose Nanocrystals (CNC) can be isolated through hydrolysis of the amorphous regions on the microfibrils by using various strong acids. The resulting crystals exhibit characteristic spatial dimensions dependent on the source of cellulose. For CNCs isolated from Microcrystalline Cellulose (MCC) length and width ranges from 35-265 nm and 3-48 nm respectively, whereas crystals, for example, isolated from cotton have a length and width of 70-300 nm and 5-15 nm. MCC, which is the choice of starting material for CNCs in this disclosure is a purified type of cellulose that have been partly depolymerized into a white, odorless crystalline powder that is insoluble in water. MCC is usually made by purifying cellulose using hydrochloric acid in a hydrolysis process, similar to the way CNCs are subsequently made from MCC, but varying in extent of depolymerization.

Cellulose Derivatives (CD) exist in many different forms, as a result of the reactivity of the hydroxyl groups on the anhydroglucose unit of cellulose, which serve as functional groups of cellulose in the sense that they are being replaced by appropriate chemical species upon derivatization.

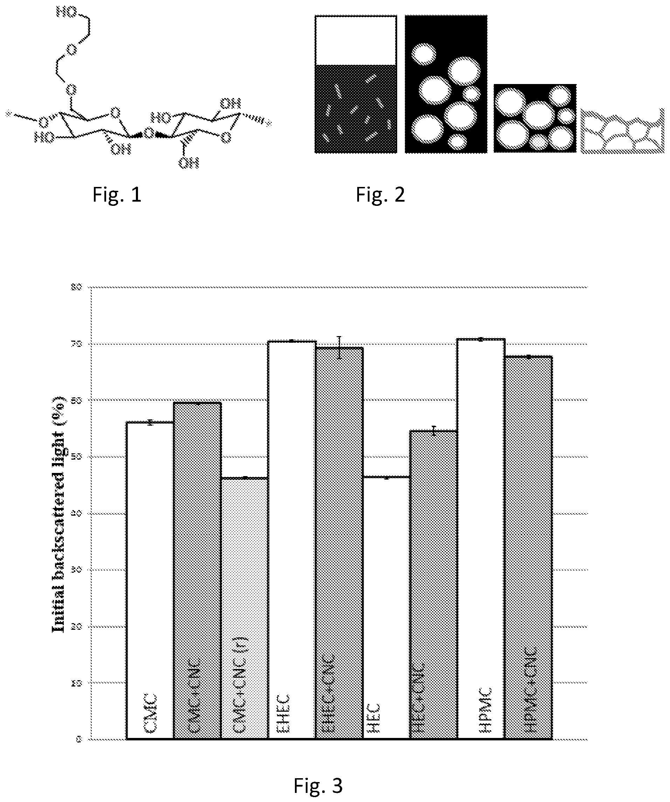

The characteristics of cellulose derivatives are as follows: i) the type(s) of chemical species substituted on the hydroxyl groups of cellulose. ii) The degree of substitution (DS), i.e. the average number of substituted hydroxyl groups per glucose unit. There are three hydroxyl groups per anhydroglucose unit, and hence the maximum degree of substitution possible is 3. iii) The molecular substitution (MS), defined as the average number of functional units present per glucose unit. For etherified celluloses this value has no theoretical limit, since reactants may add in a step-wise manner, resulting in a growing side-chain with a larger amount of functional units, hence a larger MS. iv) The molecular weight of the derivative in terms of number and weight, abbreviated $M_n$ and $M_w$ respectively. Fig. 1 illustrates characteristics ii) and iii) where cellulose has been derivatized into hydroxyethyl cellulose. The degree of substitution (DS) in this case is 0.5 since there is one substituted hydroxyl group per six total in a structure showing two anhydroglucose units (1/6*6/2). The MS of this derivative is 1 since there are two hydroxyethyl groups per two anhydroglucose units; note that the hydroxyethyl groups being attached to the same group (at the $C_6$-position) makes no difference when it comes to the magnitude of MS.

A family of CDs, mainly used in this disclosure is that of ether derivatives. Many of the cellulose ether derivatives are water soluble and exhibits significant surface activity, factors which render them appropriate in this disclosure. The reason for this is that they are with increasing surface activity more likely to be involved in the stabilization of phase interfaces, for example in emulsions. Table 2 gives example of various water soluble cellulose ether derivatives, some of which also are used within the frames of this disclosure to produce characteristic materials.

**Table 2**

| Cellulose Derivative | Abbreviation | Functional group(s) |
|---|---|---|
| Sodium Carboxymethyl Cellulose | NaCMC | $CH_2COO^-Na^+$ |
| Hydroxypropyl Methyl Cellulose | HPMC | $CH_2CH(OH)CH_3$ / $CH_3$ |
| Methyl Cellulose | MC | $CH_3$ |
| Hydroxyethyl Cellulose | HEC | $CH_2CH_2OH$ |
| Ethyl Hydroxyethyl Cellulose | EHEC | $CH_2CH_3$ / $CH_2CH_2OH$ |
| Hydroxypropyl Cellulose | HPC | $CH_2CH(OH)CH_3$ |

An emulsion refers to a system of two immiscible liquids where one of the liquids has been dispersed in the other by addition of an emulsifier. The emulsifier is generally a surface active molecule, but Ramsden and Pickering showed in the beginning of the 20th century that particles can also be used as emulsifiers to produce stable emulsions. Emulsions are categorized either as water-in-oil (w/o) or oil-in-water (o/w) emulsions, which refers to what phases are dispersed and continuous. In the case with o/w-emulsions the oil is dispersed in a continuous phase of water and vice versa for a w/o-emulsion. The character of an emulsion is strongly related to the chemistry of the emulsifier, which if relatively hydrophilic tends to produce o/w-emulsions. One example of such an emulsion is that which is produced if CNCs are used as emulsifier. The relatively hydrophilic character of CNCs makes the o/w-emulsion preferred.

In this disclosure a solid emulsion refers to a system of a solid continuous phase and a liquid dispersed phase. Solid emulsions can be produced from regular emulsions and it is then required that the emulsions do not phase separate when being processed. Processes that can be applied to transform an emulsion into a solid emulsion include solidification of continuous phase through solvent casting and polymerization of continuous phase. The process of making a solid emulsion is as follows: i) dissolution of polymer and emulsifier in aqueous phase ii) emulsification of aqueous phase with oil phase iii) evaporation of polymer solvent (water) in the continuous phase. These three steps puts requirement on the material used: i) the polymer to be used has to be water soluble. ii) The polymer solution cannot be too viscous at appropriate concentrations or it may hinder emulsification. iii) The polymer solution (with potential added emulsifier) has to be sufficiently surface active for an emulsion to form. iv) the polymer has to form a protective matrix during evaporation of the water to prevent coalescence of oil droplets. Fig. 2 is a schematic illustration of the process of making a solid emulsion through solvent casting of the continuous phase. Oil (white) is emulsified with aqueous polymer solution (black) and added emulsifier (grey), producing oil droplets. The emulsion is dried and the dissolved polymer solidifies upon the evaporation of the solvent; resulting in a solid emulsion.

EXPERIMENTS

A number of emulsions, prepared according to the method described below, were tested to assess their properties.

The CNC obtained from commercial sources was characterized by means of Atomic Force Microscopy (AFM). Pieces of mica were cut and freshly cleaved using double-sided tape. A few drops of 0.1% (w/w) cationic polyethylenimine (PEI, $M_w$ = 40000 $g/mole$) were added to the mica plate prior to the addition of CNC to ensure adhesion of the negatively charged specimens to the plate. The mica plate containing PEI were dried with nitrogen after which a few drops of a 0.05% (w/w) CNC-suspension was added and dried with nitrogen in a similar manner. The PEI and CNC-suspension were let to rest only one minute before drying with nitrogen to ensure no aggregation would occur, that if present would make sizing difficult. AFM was performed (NT-MDT Atomic Force Microscope) using silicon cantilevers with a golden reflective side and a force constant of 1.45-15.1 N/m in a semi-contact mode. Micrographs were analyzed to determine dimensions of the isolated nanocrystals..

**Emulsion preparation**

CDs used for solid emulsions were diluted in Milli-Q (18.2 MΩ) to manageable viscosities that wouldn't hinder emulsification, usually corresponding to a solution of 4-7% (w/w), depending on the type of CD. The actual concentration of the CDs was measured gravimetrically after drying solutions in an oven to get a more accurate value of the solid content.

Emulsions were prepared by adding CD solution to a plastic vial (50 ml) containing the CNC-suspension. Calcium chloride was then added together with additional Milli-Q (18.2MΩ) to meet the desired oil/water ratio. The oil phase was added to the continuous water phase, after which emulsification and homogenization was performed using either a Diax 900 homogenizer (Heidolph Instruments) or an UltraTurrax IKA T25 digital at a speed of 20000-23000 rpm. Emulsions No. 27-34 were made in a Heidoph Silent Crusher M, dispersion tool 22F. The resulting emulsion was homogenized for 5 minutes, and was then left for 1 week.

The components used for preparation of the emulsions are disclosed in Table 3. 36 samples were prepared, having the compositions shown in Table 4. The emulsions were stored in room temperature and the emulsion stability was evaluated after 1 week.

**Table 3**

| Oils | Isopropyl palmitate, technical grade 90%, Aldrich; 2-ethylhexyl palmitate, Crodamol OP-LQ-(RB), Croda; |
|---|---|
| | Olive oil, pure, virgin, Acros; |
| | Liquid Paraffin, MedicWay 68, Statiol Sweden; Tween 80-LQ-(CQ); |
| | Dodecane Reagentplus, >99%, Sigma Aldich; |
| | Liquid Paraffin Ondina X430 E-1002644 |
| | Silicone oil, Wacker AK 350 Silicone Fluid |
| CNC | Cellulose NanoCrystaline, Celluforce NCC; |

(continued)

| Cellulose derivatives (CD) | CMC, Gabrosa ds=0.56, v=121; |
| | Carboxymethyl cellulose sodium salt (Sigma Aldrich) Mw=90000; d=1.59; |
| | HPMC 60SH-50 50cP type 2910 (Shiu ETSU Chemical); |
| | HPC-SSLMw=40000 (Nisso Chemical) HEC 250 G PHARM Natrosol sample; |
| | EHEC Bermocoll E230 FQ (Akzo Nobel); CaCl2, Merck. |
| | HEC 250 HHX PHARM Natrosol. EHEC Bermocoll E230 FQ (Akzo Nobel) |
| | MC (Methyl Cellulose) Methocel A4M Premium (Dow Chemicals) |

Other materials used in experiments were Hexadecane Reagentplus >99% (Sigma Aldrich); Fluorescein isothiocyanate isomer I (Sigma Aldrich).

**Table 4**

| Sample No. | CD | CD wt% | CNC wt% | CaCl wt% | Water wt% | Oil | Oil wt% | Solid Content Wt% | Emulsion Stability after 1 week |
|---|---|---|---|---|---|---|---|---|---|
| 1 | EHEC | 2 | 0.5 | 0.033 | 72.5 | 2-ethylhexyl palmitate | 25 | 2,5/27,5 | Ok |
| 2 | HPMC | 2 | 0.5 | 0.033 | 72.5 | 2-ethylhexyl palmitate | 25 | 2,5/27,5 | Ok |
| 3 | HEC | 2 | 0.5 | 0.033 | 72.5 | 2-ethylhexyl palmitate | 25 | 2,5/27,5 | Ok |
| 4 | CMC | 2 | 0.5 | 0.033 | 72.5 | 2-ethylhexyl palmitate | 25 | 2,5/27,5 | Ok |
| 5 | NaCMC | 2 | 0.5 | 0.033 | 72.5 | 2-ethylhexyl palmitate | 25 | 2,5/27,5 | Ok |
| 6 | HEC | 0.5 | 2 | 0,033 | 72.5 | 2-ethylhexyl palmitate | 25 | 2,5/27,5 | Ok |
| 7 | HPMC | 0.5 | 2 | 0,033 | 72.5 | 2-ethylhexyl palmitate | 25 | 2,5/27,5 | Ok |
| 8 | EHEC | 2 | 0.5 | 0.033 | 72.5 | Dodecane | 25 | 2,5/27,5 | Ok |
| 9 | EHEC | 2.5 | - | | 72.5 | Dodecane | 25 | 2,5/27,5 | Ok |
| 10 | HPMC | 2 | 0.5 | 0.033 | 72.5 | Dodecane | 25 | 2,5/27,5 | Ok |
| 11 | HPMC | 2.5 | - | | 72.5 | Dodecane | 25 | 2,5/27,5 | Ok |
| 12 | HEC | 2 | 0.5 | 0.033 | 72.5 | Dodecane | 25 | 2,5/27,5 | Ok |
| 13 | HEC | 2.5 | - | | 72.5 | Dodecane | 25 | 2,5/27,5 | Not Ok |
| 14 | CMC | 2 | 0.5 | 0.033 | 72.5 | Dodecane | 25 | 2,5/27,5 | Ok |
| 15 | CMC | 2.5 | - | 0.033 | 72.5 | Dodecane | 25 | 2,5/27,5 | Ok |
| 16 | HEC | 2 | 0.5 | | 72.5 | Olive oil | 25 | 2,5/27,5 | Ok |

(continued)

| Sample No. | CD | CD wt% | CNC wt% | CaCl wt% | Water wt% | Oil | Oil wt% | Solid Content Wt% | Emulsion Stability after 1 week |
|---|---|---|---|---|---|---|---|---|---|
| 17 | HPMC | 2 | 0.5 | | 82.5 | Olive oil | 15 | 2,5/17,5 | Not Ok |
| 18 | HPMC | 2 | 0.5 | | 62.5 | Olive oil | 35 | 2,5/37,5 | Ok |
| 19 | HPMC | 2 | 0.5 | | 72.5 | Liquid Paraffin | 25 | 2,5/27,5 | Ok |
| 20 | HEC | 2 | 0.5 | | 82.5 | Liquid Paraffin | 15 | 2,5/17,5 | Ok |
| 21 | HEC | 2 | 0.5 | | 62.5 | Liquid Paraffin | 35 | 2,5/37,5 | Ok |
| 22 | HEC | 1 | 0,25 | 0,033 | 73,75 | Isopropyl Palmitate | 25 | 1,25/26,25 | Ok |
| 23 | HPMC | 1 | 0,25 | 0,033 | 73,75 | Isopropyl Palmitate | 25 | 1,25/26,25 | Not Ok |
| 24 | EHEC | 1 | 0,25 | 0,033 | 73,75 | Isopropyl Palmitate | 25 | 1,25/26,25 | Not Ok |
| 25 | CMC | 1 | 0,25 | 0,033 | 73,75 | Isopropyl Palmitate | 25 | 1,25/26,25 | Ok |
| 26 | NaCMC | 1 | 0.25 | 0.033 | 73,75 | Isopropyl Palmitate | 25 | 1,25/26,25 | Ok |
| 27 | CMC | 2 | 0.5 | | 77,5 | Liquid Paraffin | 20 | 2,5/22,5 | Ok |
| 28 | HPMC | 2 | 0.5 | | 77,5 | Liquid paraffin | 20 | 2,5/22,5 | Ok |
| 29 | HEC | 2 | 0.5 | | 77,5 | Liquid Paraffin | 20 | 2,5/22,5 | Not Ok |
| 30 | HEC/HPMC | 2 | 0.5 | | 77,5 | Liquid paraffin | 20 | 2,5/22,5 | Ok |
| 31 | CMC | 2 | 0.5 | | 77,5 | Silicone Oil | 20 | 2,5/22,5 | Not Ok |
| 32 | HPMC | 2 | 0.5 | | 77,5 | Silicone Oil | 20 | 2,5/22,5 | Ok |
| 33 | HEC | 2 | 0.5 | | 77,5 | Silicone Oil | 20 | 2,5/22,5 | Not Ok |
| 34 | HEC/HPMC | 2 | 0.5 | | 77,5 | Silicone Oil | 20 | 2,5/22,5 | Ok |

Emulsions No. 1-5 were applied to five different substrates, with the emulsion in wet condition, by placing the emulsion prepared as described above between two rollers, and then pull rollers over the substrate twice. The substrates were round pieces having a diameter of 60 mm, samples were punched out using an Atom S.p.A from the materials disclosed in Table 5.

**Table 5**

| Substrate | |
|---|---|
| PP | polypropylene nonwoven,16 gsm, FIBERWEB Biesheim, Id No. B4415402100% syntetiska fibrer |
| PET | TWE (fd Libeltex, Belgien) 80 gsm, Composition 35% 12 den, PET35% 6 den PET/PET (whereone of the PET has lower melting point), 30% 3 den bico PP/PET |

(continued)

| Substrate | |
|---|---|
| Tissue Nonwoven | Tork, Cleaning Cloth, 510178, by SCA ca. 35% Synthetic fiber+65% cellulose fiber |
| Tissue | Tempo, cotton touch, 4 plies, 100% cellulosa fibrer tre lager |
| PUR | PUR (Polyurethane) foam from Caligen E50 |

The wet uptake, i.e. the weight of emulsion held by the substrate before drying of the emulsion was determined by weighing the substrate before and after application of the emulsion was according to Table 6 (three samples per formulation).

**Table 6**

| | | Wet uptake (g emulsion/g substrate) | | | | |
|---|---|---|---|---|---|---|
| Emulsion No. (Table 5.2) | | 1 | 2 | 3 | 4 | 5 |
| CD | | EHEC | HPMC | HEC | CMC | NaCMC |
| Substrate | PP | 0,09 | 0,48 | 0,07 | 0,14 | 0,16 |
| | PET | 0,01 | 0,03 | 0,01 | 0,02 | 0,02 |
| | Tissue | 0,28 | 0,24 | 0,11 | 0,11 | 0,10 |
| | Tissue Nonwoven | 0,06 | 0,39 | 0,03 | 0,06 | 0,09 |
| | Skum PUR | 0,02 | 0,17 | 0,10 | 0,09 | 0,09 |

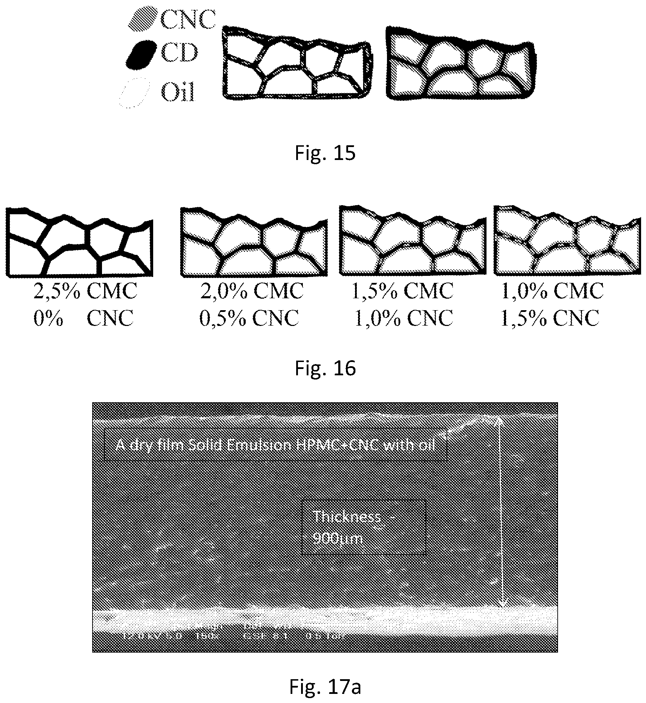

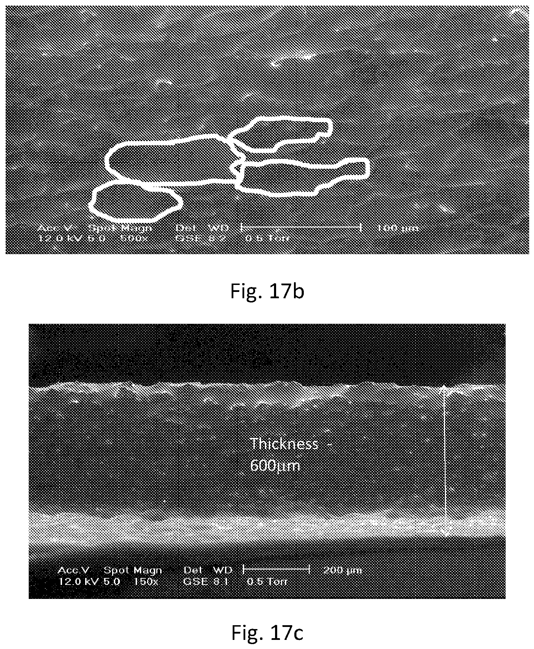

Fig. 17-23 show ESEM (Environmental Scanning Electron Microscope) images of solid emulsions and substrates carrying solid emulsion, where the emulsion has been allowed to dry and form a solid continuous phase with liquid phase dispersed therein. The drying was done at room temperature at 23C and 50% RH.

Fig. 17a shows a solid emulsion specimen of emulsion No.2, before compression, having a thickness of 900 $\mu$m. Fig. X.1b is an enlargement of the center portion of Fig. X.1a, in which oil phase drops are highlighted by means of white lines. Fig. X.1c is the same specimen after compression to a thickness of 600 $\mu$m. No oil phase drops can be seen, since the oil phase has been released.

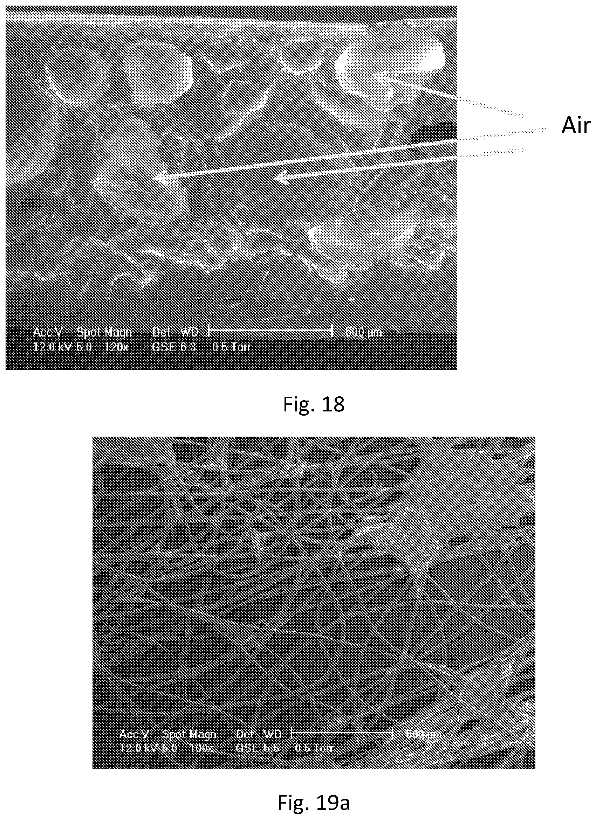

Fig. 18 shows a foamed specimen of the same solid emulsion (No.2). Air filled pores are highlighted by means of arrows.

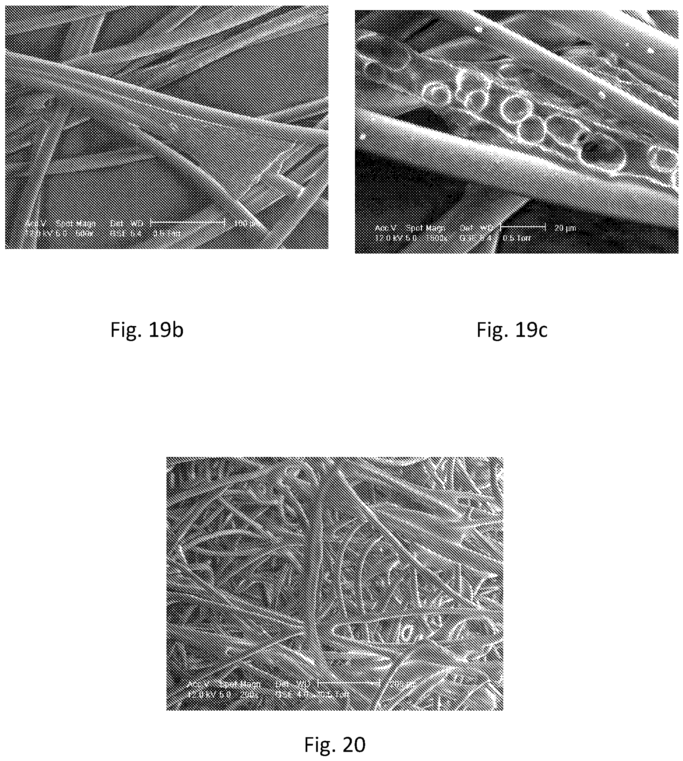

Fig. 19a-c are images of a PP nonwoven (Table 5) treated with solid emulsion (SE), where the wet uptake is 0,48 g SE/g substrate. It can be seen that the solid emulsion is incorporated in the structure, on fibers, at fiber crossings and/or at the binding points. Fig. 19c shows how the solid emulsion is holding oil.

Fig. 20 is an image of a PET nonwoven (Table 5) treated with solid emulsion (No.2), where the wet uptake is 0,03 g SE/g substrate.

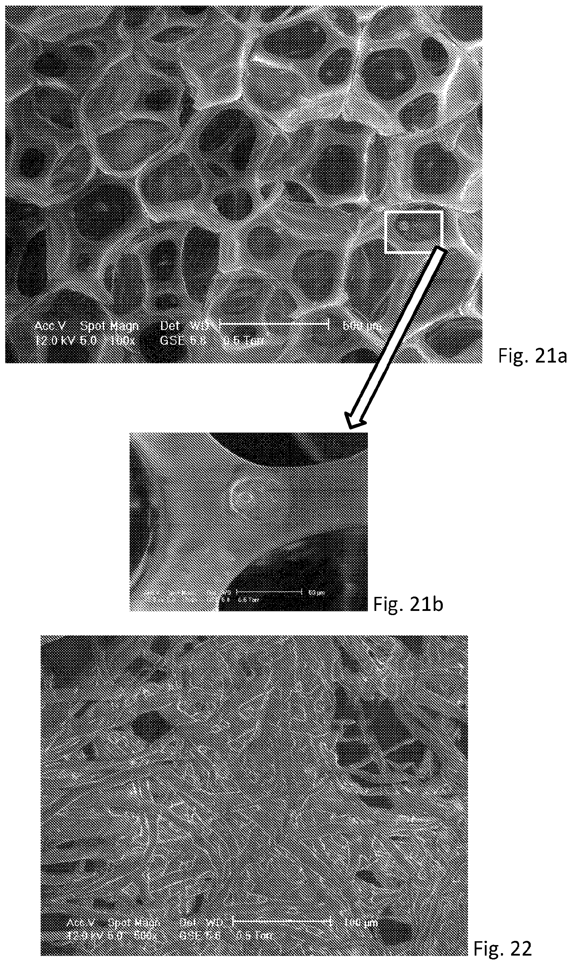

Fig. 21a is an image of a PUR foam (Table 5) treated with solid emulsion (No.2), where the wet uptake is 0,17 g SE/g substrate. The enlarged image in Fig. 21b shows how there is solid emulsion present of the cell wall.

Fig. 22 is an image of a tissue (Table 5) treated with solid emulsion (No.2), where the wet uptake is 0,24 g SE/g substrate.

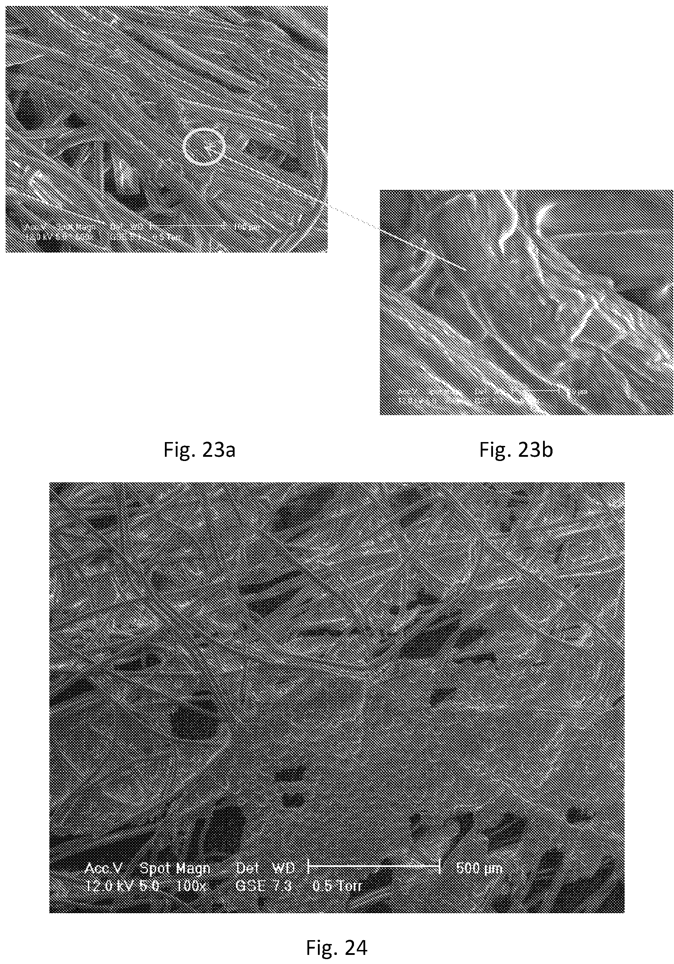

Fig. 23a-b are images of a tissue nonwoven (Table 5) treated with solid emulsion (No.2), where the wet uptake is 0,39 g SE/g substrate.

Fig. 24 is an image of a PP nonwoven (Table 5) treated with an excess of solid emulsion (No.2).

Emulsions No. 8-15 were analyzed by means of various methods described below.

Light microscopy was performed by adding a few drops of fresh emulsion into a plastic vial, after which the emulsion was diluted with appropriate amount of distilled water. The amount of water used was determined visually based on the turbidity so that the emulsion droplets would be distinguishable whilst still showing a statistically relevant amount of droplets. The size of the emulsion droplets was determined using a shape recognizing software which was tuned to identify the majority of the droplets present in each micrograph. No requirements on perfect recognizing was made, instead it was made sure that the program didn't identify random non-droplet artifacts as droplets.

The choice of cellulose derivatives was based on their performances as additives in emulsion formulations with CNCs. A summary of studied CDs can be seen in table 7 where their grade and viscosity indicated what CD it was. Performances were based on visual observation where an unstable or unsuitable emulsion simply was referred to as

"-". Some emulsions were seemingly stable in the wet state but not during drying and was therefore also referred to as "-" or "+" in the solid emulsion column.

**Table 7**

| CD | Viscosity (mPas (2%)) | Emulsion (wet) | Solid emulsion |
|---|---|---|---|
| MC | 4000 [30] | + | - |
| HPMC | 50 [33] | + | + |
| HPMC | 100 [33] | + | + |
| HEC HHX | 4500* [31] | - | - |
| HEC G | 250-400 [31] | + | + |
| CMC | 50-200** [32] | + | + |
| EHEC | >400 | + | + |
| HPC-SSL | <50 [34] | + | - |
| *viscosity measured in a 1% solution. **viscosity measured in a 4% solution | | | |

Emulsions No. 8-15 were analyzed Multiple Light Scattering within 10 minutes of production by pouring fresh polymer pickering emulsions in customized tubes that were then inserted in a Turbiscan MA2000. The magnitude of backscattered light, both initial and time dependent, were collected over a specific length interval in the tube corresponding to data points ranging from 600 to 1400 out of 1800 total points. The length interval was chosen to make sure variations in light intensity were meaned over a suitable set of data points. Initial magnitude of backscattered light was collected to get information regarding the average relative droplet size in the emulsions, whereas time dependent (over 24 hours) were means to assess the relative stability of the emulsions.

Solid emulsions were prepared from emulsions No. 8-15, by pouring the emulsions into petri dishes and allowing them to dry. The amount of time required for an emulsion to dry was tracked gravimetrically where a steady state occurred where then all of the water had been assumed to have left the emulsion since the mass ratio did not change as a function of time. Solid emulsions assumed to be ready for characterization when the mass ratio in the petri dish did not change as a function of time. The mass of the individual emulsions were always 20 grams.

The solid emulsions thus obtained were characterized by means of Confocal Laser Scanning Microscopy (CLSM) and Mechanical Testing. The solid emulsions used in the CLSM were as described above, but with addition of 10-100 ppm Fluorescein isothiocyanate (FITC) to the water phase prior to emulsification. The addition of FITC was performed to make the solid structure of the solid emulsion fluoresce and hence become more easily visible and distinguished from the oil in the emulsion. Emulsions that were analyzed in CLSM were dried on the glass plates that were used during microscopy. This was done to not affect the microstructure by cutting or compressing during sample preparation. A small amount of emulsion was analyzed during CLSM so drying was not required to be continued by the same amount of time as with the emulsions in petri dishes.

For mechanical testing, solid emulsion samples were punched from casted films to produce circular discs with a diameter of 16 mm and characteristic height according to that of the solid emulsion, generally varying between 1-2mm. The height of the solid emulsion samples were determined by quadruple measurements using a digital caliper. The fresh circular discs were wiped gently to remove any oil that leaked during sample preparation and then weighted. The discs were then inserted in a universal testing machine (Instron). Compression was done with a loading cell of 5kN. The tested sample was compressed between two flat steel surfaces, which were moved towards each other at a rate of (compression rate) of 0.1% of the initial distance between the plates per second. The starting distance was set as when a normal initial force reached 10N. Multiple discs (≥3) were punched from one emulsion to give multiple measurements for each type of emulsion to ensure statistical accuracy. The solid emulsion discs were thoroughly wiped after compression before weighting to get a rough, yet sufficient estimation of the oil content. To later correlate oil leakage to volume fraction of oil in the initial solid emulsion equation 3.1 was used, which required the density of dispersed and continuous phase and the mass fraction of leached oil.

$$\phi_{dispersed} = \frac{(m_{leached} - 1)\rho_{dispersed}}{m_{leached} \cdot \rho_{continious}} + 1$$

3.1

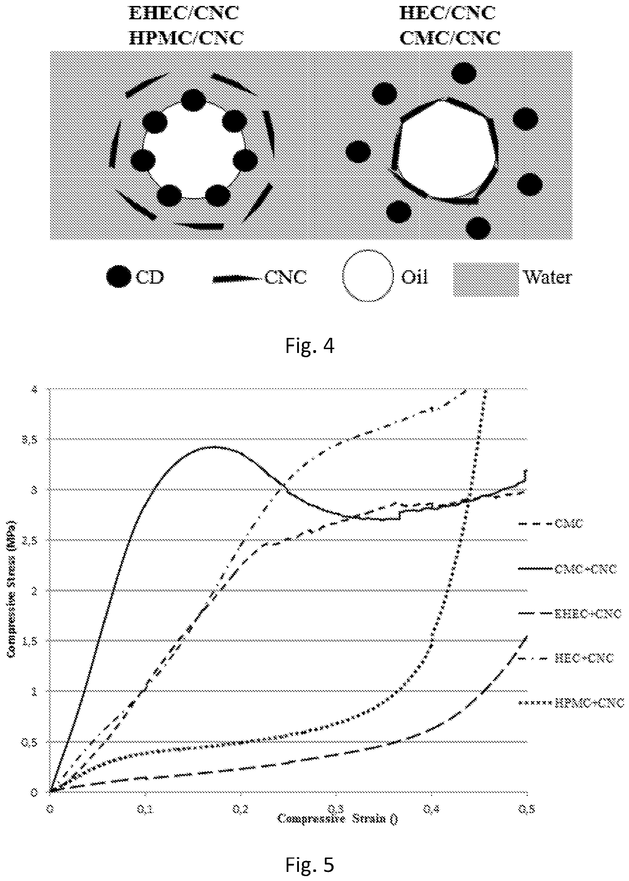

The pressure measurements (mechanical compression testing) were performed using a INSTRON instrument model/serial No: 5565AK7508 and the static load cell used for the experiments had a serial No: 66822. The "upper punch" (max load 10kN) used for the compression had the number T1223-1021 and the "lower punch" (max load 100kN) had the number of T489-74. The sample punch had a defined geometry which gave the samples a certain area which the software used to calculate pressure from the applied load. The thickness was determined since the experiments were run to compress a certain % of the thickness over time, independent on the measured thickness of the sample. The test result is shown in Fig. 5 and Table 10.

This mechanical testing can be done with the solid emulsion incorporated in a substrate and for sake of simplicity the critical pressure value is taken as 0.1 kPa.

Characterization of CNC-CD interactions for by means of UV/VIS-spectroscopy was performed (Spectrophotometer HP8453) by adding $2000\mu L$ CNC 0.3% or 0.6% suspension to quartz cuvettes. CDs were then added from a solution with similar concentration as that of the CNC suspension. Measurements were done after each incremental addition of derivative until the final ratio of CD to CNC was 0.5. Distilled water was used as blank for all measurements. Concentrations of the species in the cuvettes were kept low to hinder viscous effects that would result in dispersion of air bubbles in the cuvettes which would give rise to inaccurate absorbance values.

Surface tension of relevant CDs were measured using the Du Noüy ring method (Sigma 60 tensiometer) on solutions containing 0.3 wt % CDs. Final values of surface tensions were automatically calculated through the mean value of five measurements in series per CD. Accuracy of obtained values were furthermore determined through earlier measurements on pure water which was compared with theoretical values.

In a characterization of nanocellulose, microfibrillated cellulose (MFC), was used as a substitute or alternative to CNCs as emulsifier in the emulsions in this disclosure. MFC was for these reasons analyzed with AFM and compared with CNCs. Results visualized the hierarchical structure of cellulose, where elementary fibrils with amorphous and crystalline regions, approximately 40 nm wide, were found to originate from larger microfibrils approximately 150 nm wide. Also notable were the extremely long fibrils of MFC (relative to CNCs), which can affect the applicability as emulsion stabilizers.

The studied emulsions contain 2.5% solid content: 2.0% CD + 0.5% CNC or 2.5% CD. This specific concentration was based on the choice of CD, where it was found that appropriate CD were not too viscous during emulsification whilst still estimated to be able to form a solid emulsions with sufficient amount of solid content.

Assessment of emulsion stability using Multiple Light Scattering (MLS) reveals significant differences in initial oil droplet size amongst the emulsions with varying type of CDs. Emulsions produced with EHEC (No. 8-9) and HPMC (No. 10-11) exhibited smaller droplet sizes compared to those with HEC (No. 12-13) and CMC (No. 14-15), whereas EHEC and HPMC relative to each other were quite similar (68-70% Backscattered light (BS)). These emulsions only showed minor oil droplet size change (decline) with the addition of CNCs; indicating that the polymers played a major role in characteristic oil droplet size. For emulsions with HEC and CMC the trend was the opposite, though minor in the case of CMC; indicating that the CNCs were to a greater extent involved in the oil droplet character. Fig. 3 shows the magnitude of the initial backscattered light of emulsions with given composition of CDs and CNCs with a dodecane content of 25% wt. Also shown is the backscattered light of a redispersed CMC/CNC emulsion. Data was collected less than 10 minutes after emulsification. In order to characterize the relative stability of emulsions with varying CDs with and without CNCs, MLS was performed over 24 hours. Stability in this section refers to small amount of coalescence as indicated by small decrease in intensity of backscattered light over time. The relative stability of emulsions solely made with CDs ranked in the following order (see table 4.1), from the most stable to the least; CMC(1.1%)>EHEC(2.8%)>HPMC(4.1%)>HEC(46.3%). Emulsions with HEC showed to be largely phase separated after 24 hours; an observation which was not found in any of the other emulsions. The phase separation of emulsions solely stabilized with HEC in relation to the stability of the ones with HEC + CNCs shows interesting contrasts, which points on the role of the CNCs; to be active at the oil/water interface. It has from this stability test been shown that HEC is an extremely poor emulsifier and that the polymer is in no way involved in the character of the oil droplets for emulsions with HEC and CNCs.

With the addition of CNCs (keeping the solid content constant) the stability ranking changed to CMC(0.6%)>HEC(1.9%)>EHEC(2.9%)>HPMC(5.6%). The stability of CMC and HEC emulsions were found to be enhanced by the addition of CNCs, though the relative enhancement of CMC emulsions was insignificant in comparison to HEC emulsions. HEC went from being completely unstable (46% decrease in BS) to one of the most stable (2% decrease in BS) with the addition of CNCs. The stability of EHEC was unaffected by the addition of CNCs with its minor change of 0.1% BS, whereas the one with HPMC decreased as indicated by the increase in difference from 4.1% to 5.6%.

The most stable emulsions were produced with CMC, and although CMC on its own produced extremely stable emulsions (2nd in stability rankings) it was shown that CNCs could enhance the stability even further (from 1.1% BS to 0.6% BS). In the case of CMC, electrostatic interactions have be acknowledged and credited for the unique stability. These aspects become even more interesting in the case of CMC and CNCs, given that the CNCs are negatively charged just as the polymeric part of CMC, and the stability that emulsions with these components show is on another level relative to other emulsions in this disclosure. **Table 8** shows the difference in BS light over a time period of 24 hours for emulsions with given composition of CDs and CNCs. All these emulsions were made with 25% dodecane. A large value in difference in BS over measured time period corresponds to a relatively unstable emulsion.

**Table 8**

| Emulsion No. | Emulsion Composition | △BS |
|---|---|---|
| 15 | 2.5% CMC | 1.07±0.2 |
| 14 | 2% CMC 0.5% CNC | 0.57±0.1 |
| 9 | 2.5% EHEC | 2.82±0.2 |
| 8 | 2% EHEC 0.5% CNC | 2.92±0.4 |
| 13 | 2,5% HEC | 46.3±0.2 |
| 12 | 2% HEC 0.5% CNC | 1.87±0.7 |
| 11 | 2,5% HPMC | 4.08±0.1 |
| 10 | 2% HPMC 0.5%CNC | 5.57±0.3 |

The usage of HPMC and EHEC were found to form small droplets. This character persisted after the substitution of 20% of the CDs with CNCs, resulting in no significant influence of CNCs for emulsions with HPMC and EHEC. Emulsions with only CMC and HEC exhibited a character of large droplets. These large droplets were affected by the substitution of 20% of the CDs with CNCs in the sense that smaller droplets were obtained, resulting in significant influence of CNCs for emulsions with CMC and HEC. For these reasons it must be that CNCs were always responsible for the droplet character if they were present in emulsions with CMC and/or HEC. The contrast in character between emulsions containing EHEC/HPM and CMC/HEC, respectively is illustrated in Fig. 4, which is an illustration of the deduced character of emulsions droplets with different CDs.

Five types of emulsions that produced stable solid emulsions were in the following sections subjected to mechanical and structural characterization, to distinguish them in relation to varying CDs. One series of the top performing solid emulsion was also mechanically tested with a varied ratio of CNC/CD.

Four solid emulsions with a theoretical (ideal) constant solid content of 9.1% and CD/CNC mass ratio of 4 with variable type of CDs were mechanically compressed. The one emulsion without CNCs (2.5% CMC, 9.1% Solids) was also added in the compression tests. Large contrasts between mechanical properties (expressed as E-moduli and compressive strength) were found between HPMC/EHEC and HEC/CMC emulsions. The compressive E-modulus was for all emulsions calculated as the slope between compressive stress and strain at 10% strain. Chosen strain of 10% was based on the observation that tested emulsions exhibited a rather linear stress-strain relation within that interval. The physical quantity defined as "compressive strength" or "energy required to compress" was calculated as the area under the stress-strain curve.

Emulsions No. 27-34 were also mechanically tested.

**Table 9**

| Emulsion No. | CD/CNC/Oil | Stability |
|---|---|---|
| 29 | CMC+CNC+Paraffin | stable, cohesive material |
| 30 | HPMC+CNC+Paraffin | stable, cohesive material |
| 31 | HEC+CNC+Paraffin | Unstable after emulsification and/or during drying |
| 32 | HEC/HPMC+CNC+Paraffin | stable, but not a cohesive material suitable for testing |
| | | |
| 33 | CMC+CNC+Silicone | unstable (more tests pending on different grade CMC) |
| 34 | HPMC+CNC+Silicone | stable, cohesive material |
| 35 | HEC+CNC+Silicone | unstable |
| 36 | HEC/HPMC+CNC+Silicone | stable, cohesive material |

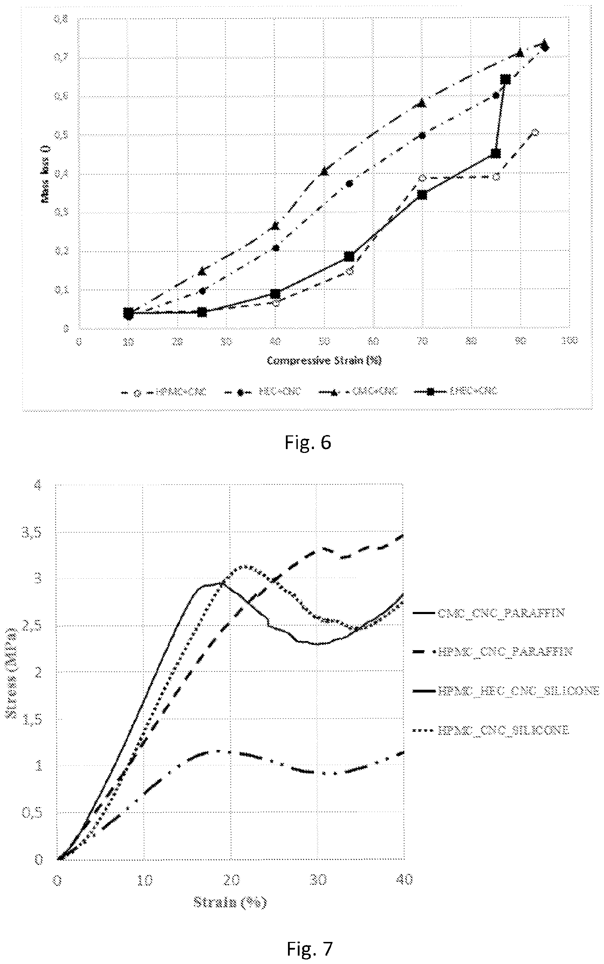

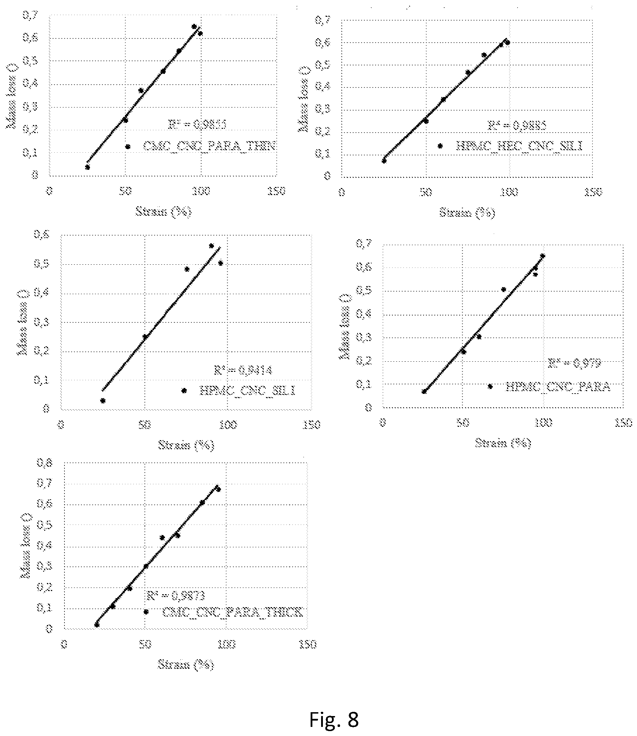

Fig. 7 shows stress-strain curves and Fig. 8 shows mass loss (oil leakage), as a function of compressive strain. The solid emulsion with a mixture of HEC/HPMC is significantly softer than the rest, whilst containing around equal amounts of oil, which is a good indication on mechanical diversity. Each curve was obtained from a mean-value from eight different samples.

E-moduli for solid emulsions made with EHEC/CNCs and HPMC/CNCs were found to range between 1 and 4 MPa, whereas those with CMC/CNCs and HEC/CNCs had E-moduli of 30 and 10 MPa respectively. The E-modulus for solid emulsions solely made with CMC was found to have approximately the same value as HEC/CNCs on 10 MPa. The required energy to compress given emulsions 50% ranged from 0.2 and 0.6 MPa for EHEC and HPMC to 1.4 and 1.3 MPa for CMC and HEC. See table 10 and Fig. 5 for values and stress-strain curves for solid emulsions. E-modulus is a measure of stiffness and as such it can be perceived that the (compressive) stiffness of the solid emulsions ranked in the following order: CMC/CNCs > > CMC HEC/CNCs > > HPMC/CNCs > EHEC/CNCs.

Energy required to compress the solid emulsions (strength) is directly related to the inherent mechanical force of which the solids emulsions are able to resist compressive deformation. This capacity ranked in the following order: CMC/CNCs > HEC/CNCs > CMC > HPMC/CNCs > EHEC/CNCs. A difference can be observed when comparing stiffness and strength. It can be seen that HEC/CNCs had a similar capacity to resist deformation as CMC/CNCs despite only having stiffness similar to that of CMC, both of which had a significantly lower stiffness relative to CMC/CNCs. This difference can be understood by looking at the stress-strain curve for HEC/CNCs (Fig. 5) where it is apparent that said solid emulsion do not exhibit a sudden "dip" in compressive stress at a certain strain value (see curve at strain values between 0.1 and 0.2), causing them to be able to absorb larger amount of energy despite being less stiff. This "dip" is referred to as (compressive) yield strength and was only exhibited by solid emulsions with CMC/CNCs. The apparent yielding of the CMC/CNC-solid emulsion was not seen as an indication of material failure as the rate of oil leakage from the emulsion in relation to strain remained constant.

The stress-strain curves for HPMC/CNCs and EHEC/CNCs show their relatively poor mechanical properties; they are neither stiff nor strong. Stress only goes up upon passing strain values at 0.4-0.5 and this is just confirmed as a consequence of the crushing of the material at which point the solid emulsions show increased stress response due to the close packing of the solids in the material. This increase in stress at large strain values was not seen as a material property, and is the reason why strain value above 50% was not included in this part. Fig. 5 shows the compressive stress vs strain relation of solid emulsions made with 0.5% CNCs and four different CDs (2.0%). Data for one CMC-emulsion without CNCs (2.5% CMC) also shown. Standard deviations are plotted as dashed lines. Notable features are the low amount of stress required to induce given strain in solid emulsions made with HPMC/CNC and EHEC/CNC relative to the other solid emulsions.

Table 10 Compressive E-modulus and energy required to compress given specimen by 50%. Specimen denoted by their respective composition prior to drying.*Energy calculated relative to the unitless compressive strain that ranged from 0 to 0.5.

| Emulsion No. | Specimen | Energy 50% strain (MPa)* | E-modulus (MPa) |
|---|---|---|---|
| 14 | 2% CMC 0.5% CNC | 1.37±0.08 | 30.0±1.4 |
| 15 | 2.5% CMC | 0.89±0.14 | 10.6±4.4 |
| 8 | 2% EHEC 0.5% CNC | 0.20±0.05 | 1.5±0.4 |
| 10 | 2% HPMC 0.5% CNC | 0.59±0.11 | 3.85±0.2 |
| 12 | 2% HEC 0.5% CNC | 1.30±0.06 | 10.21±1.2 |

The solid emulsions also varied in amount of oil leaked upon compressive strain. EHEC/CNCs and HPMC/CNCs emulsion exhibited similar behavior where only a small quantity of oil leaked during early stage compression whereas CMC/CNCs and HEC/CNCs emulsions exhibited a rather linear relation between oil leakage and strain, see Fig. 6. The behavior of EHEC and HPMC emulsions was due to their poor performances as solid emulsions where some of the encapsulated dodecane had evaporated during storing, leaving behind a somewhat porous, weak material. The linear relation exhibited by CMC/CNCs and HEC/CNCs solid emulsions further showed characteristics of the solid emulsions where material failure due to compression is continuous rather than brittle, something which points on the inherent toughness of the solid emulsions. Initial dispersed volume fractions in the solid emulsions were also calculated from the relative mass fraction at maximum compression (Fig. 6). The volume of HPMC/CNC, EHEC/CNC, HEC/CNC and CMC/CNC solid emulsions were composed of at least (in the same order) 54%, 74%, 82% and 83% Dodecane. Not shown is also the volume fraction of pure CMC solid emulsions which contained at least 72% dodecane. The solid emulsion may have contained larger amount of oil since the calculation was based on the strain value at 90-95% and may as a result not have been completely drained during compression. Fig. 6 shows the relation between compressive strain and relative mass loss due to compression in solid emulsions made with 2.0% of given CD and 0.5% CNCs. Note the relatively linear relation for solid emulsions with HEC/CNCs and CMC/CNCs.

Solid emulsions consisting of CMC and CNCs were found to be the strongest and most robust material, so additional compression tests were conducted to look at the influence of CNCs on the strength of the material. Results (see table 11) indicate a decrease in strength and compressive E-modulus when keeping the solid content constant upon increasing CNC content. Both material strength and compressive E-modulus decreased for specimen with more than 0.5% CNCs, see table 11, showing the role of the matrix forming CD which cannot be substituted by CNCs if better mechanical properties is desired. The observation of superior mechanical properties for solid CMC-emulsions with 0.5% CNCs is furthermore interesting as this corresponds to the closest value of 100% coverage of CNCs of the oil droplets in the original emulsions. Both stiffness and material strength declined significantly after this point, something which points on the reinforcing effect of CNCs, but only up to the point of oil droplet coverage, after which the role of the CNCs change. Table 11 shows compressive E-modulus and energy required to compress given CMC-specimen by 50%. Specimen denoted by their respective composition prior to drying.

**Table 11**

| Specimen | Energy 50% strain (MPa)* | E-modulus (MPa) |
|---|---|---|
| 2.5% CMC 0% CNC | $1.04\pm0.14$ | $10.6\pm4.4$ |
| 2% CMC 0.5% CNC | $1.37\pm0.08$ | $30.0\pm1.4$ |
| 1.5% CMC 1% CNC | $0.88\pm0.21$ | $8.50\pm1.4$ |
| 0.5% CMC 2% CNC | $0.41\pm0.03$ | $3.72\pm0.4$ |
| *Energy calculated relative to the unitless compressive strain that ranges from 0 to 0.5. | | |

The result of decline in mechanical properties upon increasing CNC-content can also be understood by looking at the structure of CMC and CNCs respectively. CMC is a polymer that prior to drying is completely dissolved in the continuous phase, upon drying it will form a molecular homogeneous solid throughout the material and around the oil structures. CNCs on the contrary are colloids and will as such exhibit relatively large inhomogeneities in the dry state, corresponding to the crystals individual dimensions. Though CNCs are acknowledged to exhibit extremely good mechanical properties it should be pointed on that these properties refer to individual CNCs and not bulk of CNCs. When comparing CNCs and CMC as a bulk material it is obvious that the polymer is far superior in terms of mechanical properties.

From table 11 it is also apparent that data for solid emulsions solely made with CNCs are lacking. The reason for this is that such emulsions were shown to become unstable during drying, even with similar solid content as earlier mentioned solid emulsions. This was viewed as a result of lack of encapsulating material. For a liquid such as dodecane to be encapsulated from an emulsion, the solid material must form a dense film around the droplets of the oil. CNCs were obviously unsuitable for this as they were not dense enough in relation to dodecane which is a low viscous liquid ($\eta$=1.34 *mPas*) consisting of only 12 carbon atoms. The resulting leaking of dodecane from the CNC-solid material was inevitable.

The bulk homogeneity in the material was verified by simply observing the material at the physical edges. An overview CLSM-micrograph revealed oil containing structures at the very edge of the materials, indicating homogeneity amongst stable solid emulsions. Observed homogeneity amongst stable solid emulsions was seen as a result of the stability of parent emulsions which, unlike for example emulsions solely made with HEC, EHEC or HPMC (i.e. without CNC), did not suffer from instabilities such as creaming or coalescence. Stability of parent emulsion was for this reason seen as important for the characterization of the solid emulsion. A solid emulsion for example characterized by creaming would for example result in the gathering of oil droplets on top of the solid emulsion as dodecane is less dense than water, resulting in an observed microstructure that would deviate from the one observed in the bulk.

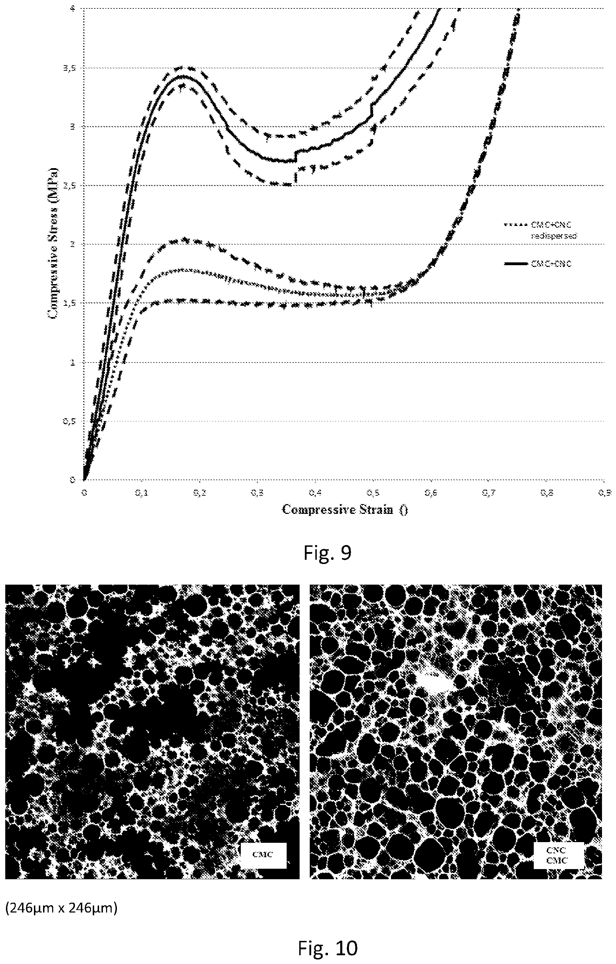

The difference between the morphology of the oil containing cells in CMC solid emulsions with and without CNCs is shown Fig. 10 where deformation of oil containing structure is apparent in the case of solid emulsions with CNCs. The specific deformed character is seen as one of the factors that makes an oil content above 74%, by volume, possible as this corresponds to maximal packing of uniform hard spheres; a condition which has been shown to be surpassed as a result of observed structure coupled with the fact that CMC/CNCs solid emulsions contain at least 83% (by volume) dispersed phase. Another property influencing the capacity for increased volume fraction dispersed phase is the polydispersity which earlier has been shown to be exhibited by produced emulsions. Polydisperse objects are more likely to pack in an efficient manner, as smaller objects can fit in between larger objects, resulting in higher capacity for dispersed phase increase. Fig. 10 shows CLSM micrographs (one focal plane) of solid emulsions made with 2.5% CMC (left) and 2.0% CMC + 0.5% CNCs (right). White feature is the cellulosic matrix with the encapsulated oil shown as black. Note the difference in morphology between the oil containing structures. Scale bars are 40$\mu$m.

The CMC/CNCs solid emulsions feature an extent of deformation of the oil containing structures. From Fig. 10 it is apparent that oil cells are connected with one another through flat interfaces made of solid CMC/CNCs, rather than circular interfaces. This flattening is seen as a result of the stability of the oil droplets which could have easily phase separated during drying, but instead it was possible for the droplets to deform into polyhedrons which are packed closely. CD/CNCs emulsions can be compared with emulsions solely stabilized by CNCs because the factor responsible for the oil character in the emulsions was largely CNCs, rather than the CDs (unless the CD was HPMC or EHEC).

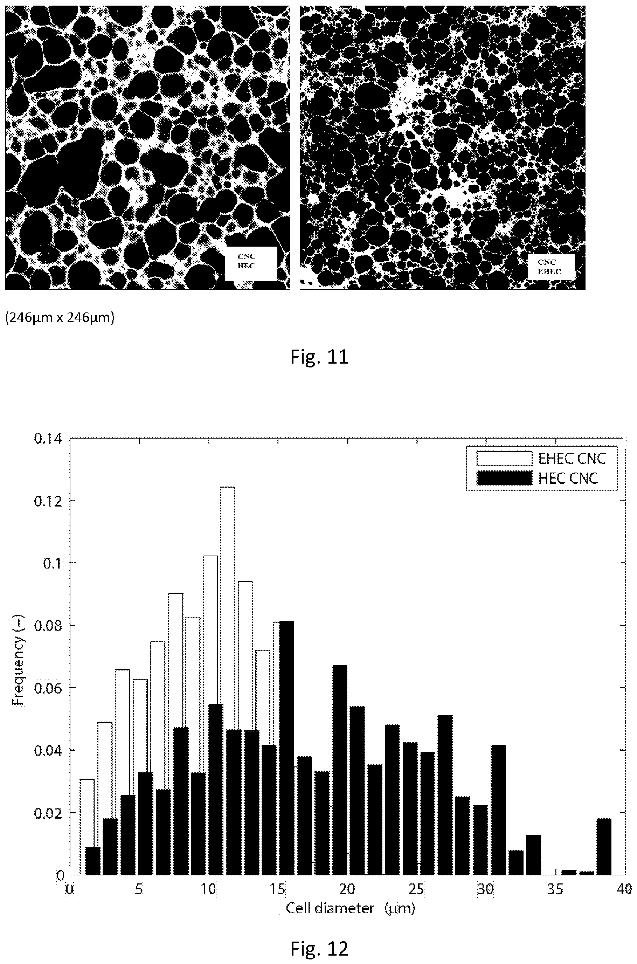

Though HEC on its own was unsuitable for emulsions and solid emulsions it was found that substitution of 20% HEC with CNCs made solid emulsions containing at least 82% (by volume) robust and stable. Results (Fig. 11) show the morphology of HEC/CNC solid emulsion. The solid emulsion was found to be similar to that of CMC/CNC, again owing its deformed character to the presence of CNCs. Solid emulsions made with EHEC/CNCs were found to exhibit a different structure, characterized by smaller, less deformed cells. The EHEC/CNC solid emulsions contained at least 74% oil (by volume). In solid emulsions with HEC/CNCs the effect of CNCs is even more pronounced. Neither CNCs nor HEC produced stable solid emulsions on their own. As previously mentioned the CNCs lacked the encapsulating properties that HEC could provide, but HEC lacked the emulsion stabilizing properties that CNCs could provide. In summary, the HEC/CNCs solid emulsion is a characteristic example of where the roles of CNCs and CDs in solid emulsions are shown. Fig. 11 shows CLSM micrographs (one focal plane) of solid emulsions made with 2.0% HEC + 0.5% CNCs (left) and 2.0% EHEC + 0.5% CNCs (right). The EHEC/CNC solid emulsions contain a large amount smaller oil containing cells relative to both HEC and CMC solid emulsions.

For EHEC/CNCs the small droplet character is, as earlier deduced, from the fact that the EHEC acts as emulsifier and stabilizer instead of the CNCs. During the drying of the emulsion the same flat interfaces as observed with CMC/CNCs and HEC/CNCs cannot be obtained since the flexibility was seen as a consequence of the CNCs, and if the CNCs are not involved in the interfaces then they cannot provide the solid emulsion with that flexibility during drying.

Fig. 12 shows the cell diameter distribution for solid emulsions with EHEC/CNCs (white) and HEC/CNCs (black).

The present disclosure also provides a method of manufacture of a fibrous layer for use as a component in the hygiene article described above. Which method comprises the steps of applying a fluid emulsion onto a web of fibrous material, said emulsion consisting of a continuous phase comprising water, cellulose derivatives and cellulose nanocrystals, and a liquid dispersed phase comprising an oil containing composition; and drying the fluid emulsion until 50-100 wt% of the water comprised in the fluid emulsion has evaporated.

A method of manufacture of the hygiene article described above comprises incorporating the fibrous material layer made as described above into a hygiene article; or applying a fluid emulsion onto a web of fibrous material already incorporated in the hygiene article, said emulsion consisting of a continuous phase comprising water, cellulose derivatives (CD) and cellulose nanocrystals (CNC), and a liquid dispersed phase comprising an oil containing composition, and drying the fluid emulsion until 50-100wt% of the water comprised in the fluid emulsion has evaporated. The fluid emulsion can be applied by printing, roll coating or spraying, slot die coating (slot extrusion), kiss-roll or roll coating, weko spraying, etc.

A method of manufacture of the hygiene article described above may alternatively comprise the steps of: preparing a film of solid emulsion by applying a fluid emulsion to a substrate; and drying the fluid emulsion until 50-100wt% of the water comprised in the fluid emulsion has evaporated, so as to obtain a solid emulsion; and removing the substrate; or preparing a foam of solid emulsion by adding a foaming agent to a fluid emulsion; applying the emulsion to a substrate; allowing the fluid emulsion to foam; drying the foamed emulsion until 50-100wt% of the water comprised in the fluid emulsion has evaporated, so as to obtain a solid foam emulsion; and removing the substrate; or preparing a solid emulsion particulate by applying a fluid emulsion to a substrate; drying the fluid emulsion until at least 50-100wt% of the water comprised in the fluid emulsion has evaporated, so as to obtain a solid emulsion; mechanically disintegrating the solid emulsion into particles; followed by incorporating the film, or foam, or particulate into a hygiene article, wherein the fluid emulsion consists of a continuous phase comprising water, cellulose derivatives (CD) and cellulose nanocrystals (CNC), and a liquid dispersed phase comprising an oil containing composition.

The continuous phase of the fluid emulsion used in the method preferably comprises 0,1-0,8 wt% cellulose nanocrystals (CNC) and 1-3 wt% cellulose derivatives (CD), based on the total weight of the fluid emulsion, and the liquid dispersed phase of the fluid emulsion is preferably comprised in an amount of 5-50 wt%, based on the total weight of the fluid emulsion.

**Claims**

**1.** A hygiene article comprising one or more layers, said article comprising a solid emulsion, which solid emulsion comprises a matrix of solid continuous phase and a dispersed oil phase, wherein the solid continuous phase comprises cellulose derivatives (CD), wherein the cellulose derivatives (CD) are chosen from carboxymethyl cellulose (CMC), hydroxyethyl cellulose (HEC), ethyl hydroxyethyl cellulose (EHEC), or hydroxylpropyl methyl cellulose (HPMC), or combinations of two or more of these cellulose derivatives, and nanocellulose, and the dispersed oil phase is an oil containing composition, wherein the solid continuous phase of the solid emulsion comprises 0,5-50 wt% nanocellulose based on the weight of cellulose derivatives (CD) comprised in the solid continuous phase.

2. The hygiene article of claim 1, wherein the solid continuous phase of the solid emulsion comprises 3-40 wt% nanocellulose, preferably 10-30 wt%, based on the weight of cellulose derivatives (CD) comprised in the solid continuous phase.

3. The hygiene article of any one of claims 1 or 2, wherein the solid emulsion comprises 50-95,5 wt% cellulose derivatives (CD) based on the total dry content weight of the solid continuous phase.

4. The hygiene article of any one of claims 1-3, wherein the weight ratio of the dispersed oil phase to the dry content in the continuous phase of the solid emulsion is 20:80-99:1 wt/wt.

5. The hygiene article of any one of claims 1-4, wherein release of the dispersed oil phase is initiated, when the a pressure above a critical pressure value is applied to the solid emulsion comprised in the article, wherein the critical pressure value preferably is 0,1 kPa.

6. The hygiene article any one of claims 1-5, wherein the solid emulsion is present in the article adhered to fibres in a fibrous layer, or in the form of a film, or a foam, or in the form of particles.

7. The hygiene article of any one of claims 1-6, wherein the article comprises two or more layers, wherein at least one layer comprises the solid emulsion, and wherein the layer comprising the solid emulsion is a fibrous layer.

8. The hygiene article of claim 7, wherein the fibrous layer is a nonwoven material, preferably comprised of hydrophobic fibres.

9. The hygiene article of claim 7, wherein the fibrous layer is a tissue layer.

10. The hygiene article of any one of claims 1-9, wherein the hygiene article is a wipe or tissue product comprising one or more layers.

11. The hygiene article of claim 10, wherein the solid emulsion is present in an amount of 0,01-20 g/m$^2$ of a layer in the product.

12. The hygiene article of claim 10 or 11, wherein the wipe or tissue product further comprises one or more intermediate layers, arranged between first and second outer layers, and wherein the solid emulsion is present in one or more of the intermediate layers.

13. A method of manufacture of a fibrous layer for use as a component in the hygiene article according to claims 1-12, comprising the steps of

- applying a fluid emulsion onto a web of fibrous material, said emulsion consisting of a continuous phase comprising water, cellulose derivatives and nanocellulose, and a liquid dispersed phase comprising an oil containing composition, and
- drying the fluid emulsion until 50-100 wt% of the water comprised in the fluid emulsion has evaporated, so as to obtain a solid emulsion, wherein the solid emulsion comprises a matrix of solid continuous phase and dispersed oil phase, the solid continuous phase comprising the cellulose derivatives chosen from carboxymethyl cellulose (CMC), hydroxyethyl cellulose (HEC), ethyl hydroxyethyl cellulose (EHEC), hydroxylpropyl methyl cellulose (HPMC), or combinations of two or more of these cellulose derivatives, and nanocellulose, the dispersed oil phase comprising the oil containing composition, and the solid continuous phase of the solid emulsion comprises 0,5-50 wt% nanocellulose based on the weight of cellulose derivatives comprised in the solid continuous phase.

14. A method of manufacture of the hygiene article of any one of claims 1-12, comprising incorporating a fibrous material layer made according to claim 13, into a hygiene article; or

applying a fluid emulsion onto a web of fibrous material already incorporated in the hygiene article, said emulsion consisting of a continuous phase comprising water, cellulose derivatives (CD) and cellulose nanocrystals (CNC), and a liquid dispersed phase comprising an oil containing composition, and drying the fluid emulsion until 50-100 wt% of the water comprised in the fluid emulsion has evaporated, so as to obtain a solid emulsion, wherein the solid emulsion comprises a matrix of solid continuous phase and dispersed oil phase, the solid continuous phase comprising the cellulose derivatives chosen from carboxymethyl cellulose (CMC), hydroxyethyl cellulose (HEC), ethyl hydroxyethyl cellulose (EHEC), hydroxypropyl methyl cellulose (HPMC), or combinations of two or more of these cellulose derivatives, and nanocellulose, and the dispersed oil phase comprising the oil containing composition, and the solid continuous phase of the solid emulsion comprises 0,5-50 wt% nanocellulose based on the weight of cellulose derivatives comprised in the solid continuous phase.

15. A method of manufacture of the hygiene article of any one of claims 1-12, comprising the steps of

- preparing a film of solid emulsion by applying a fluid emulsion to a substrate, and
- drying the fluid emulsion until 50-100 wt% of the water comprised in the fluid emulsion has evaporated, so as to obtain a solid emulsion; and
- removing the substrate;

or

- preparing a foam of solid emulsion by adding a foaming agent to a fluid emulsion;
- applying the emulsion to a substrate;
- allowing the fluid emulsion to foam;
- drying the foamed emulsion until 50-100 wt% of the water comprised in the fluid emulsion has evaporated, so as to obtain a solid foam emulsion;
- removing the substrate;

or

- preparing a solid emulsion particulate by applying a fluid emulsion to a substrate;
- drying the fluid emulsion until at least 50-100 wt% of the water comprised in the fluid emulsion has evaporated, so as to obtain a solid emulsion;
- mechanically disintegrating the solid emulsion into particles followed by
- incorporating the film, or foam, or particulate into a hygiene article,

wherein the fluid emulsion consists of a continuous phase comprising water, cellulose derivatives (CD) and nanocellulose and a liquid dispersed phase comprising an oil containing composition; wherein the solid emulsion comprises a matrix of solid continuous phase and dispersed oil phase, the solid continuous phase comprising the cellulose derivatives chosen from carboxymethyl cellulose (CMC), hydroxyethyl cellulose (HEC), ethyl hydroxyethyl cellulose (EHEC), hydroxypropyl methyl cellulose (HPMC), or combinations of two or more of these cellulose derivatives, and nanocellulose, the dispersed oil phase comprising the oil containing composition, and the solid continuous phase of the solid emulsion comprises 0,5-50 wt% nanocellulose based on the weight of cellulose derivatives comprised in the solid continuous phase .

**Patentansprüche**

1. Hygieneartikel, umfassend eine oder mehrere Schichten, wobei der Artikel eine feste Emulsion umfasst, wobei die feste Emulsion eine Matrix aus fester kontinuierlicher Phase und einer dispergierten Ölphase umfasst, wobei die feste kontinuierliche Phase Cellulosederivate (CD), wobei die Cellulosederivate (CD) aus Carboxymethylcellulose (CMC), Hydroxyethylcellulose (HEC), Ethylhydroxyethylcellulose (EHEC) oder Hydroxypropylmethylcellulose (HPMC) oder Kombinationen von zwei oder mehr dieser Cellulosederivate ausgewählt sind, und Nanocellulose umfasst und wobei es sich bei der dispergierten Ölphase um eine ölhaltige Zusammensetzung handelt, wobei die feste kontinuierliche Phase der festen Emulsion 0,5 bis 50 Gew.-% Nanocellulose bezogen auf das Gewicht der Cellulosederivate (CD), die in der festen kontinuierlichen Phase enthalten sind, umfasst.

2. Hygieneartikel nach Anspruch 1, wobei die feste kontinuierliche Phase der festen Emulsion 3 bis 40 Gew.-% Nanocellulose, vorzugsweise 10 bis 30 Gew.-%, bezogen auf das Gewicht der Cellulosederivate (CD), die in der festen kontinuierlichen Phase enthalten sind, umfasst.

3. Hygieneartikel nach einem der Ansprüche 1 oder 2, wobei die feste Emulsion 50 bis 95,5 Gew.-% Cellulosederivate (CD) bezogen auf das Gesamttrockengehaltgewicht der festen kontinuierlichen Phase umfasst.

4. Hygieneartikel nach einem der Ansprüche 1 bis 3, wobei das Gewichtsverhältnis der dispergierten Ölphase zum Trockengehalt in der kontinuierlichen Phase der festen Emulsion 20:80 bis 99:1 Gew./Gew. beträgt.

5. Hygieneartikel nach einem der Ansprüche 1 bis 4, wobei die Freisetzung der dispergierten Ölphase eingeleitet wird, wenn ein Druck über einem kritischen Druckwert auf die in dem Artikel enthaltene feste Emulsion ausgeübt wird, wobei der kritische Druckwert vorzugsweise 0,1 kPa beträgt.

6. Hygieneartikel nach einem der Ansprüche 1 bis 5, wobei die feste Emulsion in einer faserigen Schicht an Fasern haftend oder in Form einer Folie oder eines Schaums oder in Form von Partikeln in dem Artikel vorhanden ist.

7. Hygieneartikel nach einem der Ansprüche 1 bis 6, wobei der Artikel zwei oder mehr Schichten umfasst, wobei mindestens eine Schicht die feste Emulsion umfasst und wobei es sich bei der Schicht, welche die feste Emulsion umfasst, um eine faserige Schicht handelt.

8. Hygieneartikel nach Anspruch 7, wobei es sich bei der faserigen Schicht um ein Vliesmaterial handelt, das vorzugsweise aus hydrophoben Fasern besteht.

9. Hygieneartikel nach Anspruch 7, wobei es sich bei der faserigen Schicht um eine Gewebeschicht handelt.

10. Hygieneartikel nach einem der Ansprüche 1 bis 9, wobei es sich bei dem Hygieneartikel um ein Wisch- oder Gewebeprodukt umfassend eine oder mehrere Schichten handelt.

11. Hygieneartikel nach Anspruch 10, wobei die feste Emulsion in einer Menge von 0,01 bis 20 g/m$^2$ einer Schicht in dem Produkt vorhanden ist.

12. Hygieneartikel nach Anspruch 10 oder 11, wobei das Wisch- oder Gewebeprodukt ferner eine oder mehrere Zwischenschichten umfasst, die zwischen einer ersten und einer zweiten Außenschicht angeordnet sind, und wobei die feste Emulsion in einer oder mehreren der Zwischenschichten vorhanden ist.

13. Verfahren zur Herstellung einer faserigen Schicht zur Verwendung als eine Komponente in dem Hygieneartikel nach den Ansprüchen 1 bis 12, umfassend die Schritte:

> - Auftragen einer Fluidemulsion auf eine Bahn aus faserigem Material, wobei die Emulsion aus einer kontinuierlichen Phase umfassend Wasser, Cellulosederivate und Nanocellulose und einer flüssigen dispergierten Phase umfassend eine ölhaltige Zusammensetzung besteht, und
> - Trocknen der Fluidemulsion, bis 50 bis 100 Gew.-% des in der Fluidemulsion enthaltenen Wassers verdampft sind, um eine feste Emulsion zu erhalten,
> wobei die feste Emulsion eine Matrix aus fester kontinuierlicher Phase und dispergierter Ölphase umfasst, wobei die feste kontinuierliche Phase die aus Carboxymethylcellulose (CMC), Hydroxyethylcellulose (HEC), Ethylhydroxyethylcellulose (EHEC), Hydroxypropylmethylcellulose (HPMC) oder Kombinationen von zwei oder mehr dieser Cellulosederivate ausgewählten Cellulosederivate und Nanocellulose umfasst, wobei die dispergierte Ölphase die ölhaltige Zusammensetzung umfasst, und die feste kontinuierliche Phase der festen Emulsion umfasst 0,5 bis 50 Gew.-% Nanocellulose bezogen auf das Gewicht der Cellulosederivate, die in der festen kontinuierlichen Phase enthalten sind.

14. Verfahren zur Herstellung des Hygieneartikels nach einem der Ansprüche 1 bis 12, umfassend das Einbringen einer faserigen Materialschicht, hergestellt nach Anspruch 13, in einen Hygieneartikel; oder
Auftragen einer Fluidemulsion auf eine Bahn aus faserigem Material, die bereits in den Hygieneartikel eingearbeitet ist, wobei die Emulsion aus einer kontinuierlichen Phase umfassend Wasser, Cellulosederivate (CD) und Cellulosenanokristalle (CNC) und einer flüssigen dispergierten Phase umfassend eine ölhaltige Zusammensetzung besteht, und Trocknen der Fluidemulsion, bis 50 bis 100 Gew.-% des in der Fluidemulsion enthaltenen Wassers verdampft sind, um eine feste Emulsion zu erhalten, wobei die feste Emulsion eine Matrix aus fester kontinuierlicher Phase und dispergierter Ölphase umfasst, wobei die feste kontinuierliche Phase die aus Carboxymethylcellulose (CMC), Hydroxyethylcellulose (HEC), Ethylhydroxyethylcellulose (EHEC), Hydroxypropylmethylcellulose (HPMC) oder Kombinationen von zwei oder mehr dieser Cellulosederivate ausgewählten Cellulosederivate und Nanocellulose umfasst, und die dispergierte Ölphase die ölhaltige Zusammensetzung umfasst, und die feste kontinuierliche Phase der festen Emulsion umfasst 0,5 bis 50 Gew.-% Nanocellulose bezogen auf das Gewicht der Cellulosederivate, die in der festen kontinuierlichen Phase enthalten sind.

15. Verfahren zur Herstellung des Hygieneartikels nach einem der Ansprüche 1 bis 12, umfassend die Schritte:

- Herstellen einer Folie einer festen Emulsion durch Auftragen einer Fluidemulsion auf ein Substrat, und
- Trocknen der Fluidemulsion, bis 50 bis 100 Gew.-% des in der Fluidemulsion enthaltenen Wassers verdampft sind, um eine feste Emulsion zu erhalten; und
- Entfernen des Substrats;

oder

- Herstellen eines Schaums aus fester Emulsion durch Zugeben eines Treibmittels zu einer Fluidemulsion;
- Auftragen der Emulsion auf ein Substrat;
- Schäumenlassen der Fluidemulsion;
- Trocknen der geschäumten Emulsion, bis 50 bis 100 Gew.-% des in der Fluidemulsion enthaltenen Wassers verdampft sind, um eine feste Schaumemulsion zu erhalten;
- Entfernen des Substrats;

oder

- Herstellen von festen Emulsionspartikeln durch Auftragen einer Fluidemulsion auf ein Substrat;
- Trocknen der Fluidemulsion, bis mindestens 50 bis 100 Gew.-% des in der Fluidemulsion enthaltenen Wassers verdampft sind, um eine feste Emulsion zu erhalten;
- mechanisches Auflösen der festen Emulsion zu Partikeln,

gefolgt von

- Einarbeiten der Folie oder des Schaums oder der Partikel in einen Hygieneartikel,

wobei die Fluidemulsion aus einer kontinuierlichen Phase umfassend Wasser, Cellulosederivate (CD) und Nanocellulose und einer flüssigen dispergierten Phase umfassend eine ölhaltige Zusammensetzung besteht; wobei die feste Emulsion eine Matrix aus fester kontinuierlicher Phase und dispergierter Ölphase umfasst, wobei die feste kontinuierliche Phase die aus Carboxymethylcellulose (CMC), Hydroxyethylcellulose (HEC), Ethylhydroxyethylcellulose (EHEC), Hydroxypropylmethylcellulose (HPMC) oder Kombinationen von zwei oder mehr dieser Cellulosederivate ausgewählten Cellulosederivate und Nanocellulose umfasst, die dispergierte Ölphase die ölhaltige Zusammensetzung umfasst, und die feste kontinuierliche Phase der festen Emulsion umfasst 0,5 bis 50 Gew.-% Nanocellulose bezogen auf das Gewicht der Cellulosederivate, die in der festen kontinuierlichen Phase enthalten sind.

## Revendications

1. Article hygiénique comprenant une ou plusieurs couches, ledit article comprenant une émulsion solide, laquelle émulsion solide comprend une matrice de phase continue solide et d'une phase huileuse dispersée, dans lequel la phase continue solide comprend des dérivés de cellulose (CD), dans lequel les dérivés de cellulose CD) sont choisis parmi la carboxyméthylcellulose (CMC), l'hydroxyéthylcellulose (HEC), l'hydroxyéthylcellulose d'éthyle (EHEC) ou l'hydroxypropylméthylcellulose (HPMC), ou des combinaisons de deux ou plus de ces dérivés de cellulose, et la nanocellulose, et la phase huileuse dispersée est une composition contenant de l'huile, dans lequel la phase continue de l'émulsion solide comprend de 0,5 à 50 % en poids de nanocellulose sur la base de dérivés de cellulose (CD) compris dans la phase continue solide.

2. Article hygiénique selon la revendication 1, dans lequel la phase continue solide de l'émulsion solide comprend de 3 à 40 % en poids de nanocellulose, de préférence de 10 à 30 % en poids sur la base du poids de dérivés de cellulose (CD) compris dans la phase continue solide.

3. Article hygiénique selon l'une quelconque des revendications 1 ou 2, dans lequel l'émulsion solide comprend de 50 à 95,5 % de dérivés de cellulose (CD) sur la base du poids de teneur totale en matières sèches de la phase continue solide.

4. Article hygiénique selon l'une quelconque des revendications 1 à 3, dans lequel le rapport en poids de la phase huileuse dispersée sur la teneur en matières sèches dans la phase continue de l'émulsion solide est de 20/80 à 99/1% en poids.

5. Article hygiénique selon l'une quelconque des revendications 1 à 4, dans lequel la libération de la phase huileuse dispersée est initiée, lorsqu'une pression supérieure à une valeur de pression critique est appliquée sur l'émulsion solide comprise dans l'article, dans lequel la valeur de pression critique est de préférence de 0,1 kPa.

6. Article hygiénique selon l'une quelconque des revendications 1 à 5, dans lequel l'émulsion solide est présente dans l'article adhéré aux fibres dans une couche fibreuse, ou sous la forme d'un film, ou d'une mousse ou sous la forme de particules.

7. Article hygiénique selon l'une quelconque des revendications 1 à 6, dans lequel l'article comprend deux ou plus de deux couches, dans lequel au moins une couche comprend l'émulsion solide, et dans lequel la couche comprenant l'émulsion solide est un couche fibreuse.

8. Article hygiénique selon la revendication 7, dans lequel la couche fibreuse est un matériau non tissé, de préférence composé de fibres hydrophobes.

9. Article hygiénique selon la revendication 7, dans lequel la couche fibreuse est une couche de tissu.

10. Article hygiénique selon l'une quelconque des revendications 1 à 9, dans lequel l'article hygiénique est une lingette ou un produit en tissu comprenant une ou plusieurs couches.

11. Article hygiénique selon la revendication 10, dans lequel l'émulsion solide est présente en une quantité de 0,01 à 20 g/m$^2$ d'une couche dans le produit.

12. Article hygiénique selon la revendication 10 ou 11, dans lequel la lingette ou le produit en tissu comprend en outre une ou plusieurs couches intermédiaires, agencées entre des première et seconde couches extérieurs, et dans lequel l'émulsion solide est présente dans une ou plusieurs des couches intermédiaires.

13. Procédé de fabrication d'une couche fibreuse destinée à être utilisée en tant que composant dans l'article hygiénique selon les revendications 1 à 12, comprenant les étapes consistant à

- appliquer une émulsion fluide sur une toile de matériau fibreux, ladite émulsion étant constituée par une phase continue comprenant de l'eau, des dérivés de cellulose et de la nanocellulose, et une phase dispersée liquide comprenant une composition contenant de l'huile, et
- sécher l'émulsion fluide jusqu'à ce que 50 à 100 % en poids de l'eau comprise dans l'émulsion fluide se soient évaporés, de manière à obtenir une émulsion solide,
dans lequel l'émulsion solide comprend une matrice de phase continue solide et de phase huileuse dispersée, la phase solide continue comprenant les dérivés de cellulose choisis parmi la carboxyméthylcellulose (CMC), l'hydroxyéthylcellulose (HEC), l'hydroxyéthylcellulose d'éthyle (EHEC), l'hydroxypropylméthylcellulose (HPMC), ou des combinaisons de deux ou plus de deux de ces dérivés de cellulose, et la nanocellulose, la phase huileuse dispersée comprenant la composition contenant de l'huile, et la phase continue solide de l'émulsion solide comprend de 0,5 à 50 % en poids de nanocellulose sur la base du poids de dérivés de cellulose compris dans la phase continue solide.

14. Procédé de fabrication de l'article hygiénique selon l'une quelconque des revendications 1 à 12, comprenant l'incorporation d'une couche de matériau fibreux réalisée selon la revendication 13, dans un article hygiénique ; ou l'application d'une émulsion fluide sur une toile de matériau fibreux déjà incorporée dans l'article hygiénique, ladite émulsion étant constituée par une phase continue comprenant de l'eau, des dérivés de cellulose (CD) et des nanocristaux de cellulose (CNC), et une phase dispersée liquide comprenant une composition contenant de l'huile, et le séchage de l'émulsion fluide jusqu'à ce que 50 à 100% de l'eau comprise dans l'émulsion fluide se soient évaporés, de manière à obtenir une émulsion solide, dans lequel l'émulsion solide comprend une matrice de phase continue solide et de phase huileuse dispersée, la phase continue solide comprenant les dérivés de cellulose choisis parmi la carboxyméthylcellulose (CMC), l'hydroxyéthylcellulose (HEC), l'hydroxyéthylcellulose d'éthyle (EHEC), l'hydroxypropylméthylcellulose (HPMC), ou des combinaisons de deux ou plus de deux de ces dérivés de cellulose, et la nanocellulose, et la phase huileuse dispersée comprenant la composition contenant de l'huile, et la phase continue solide de l'émulsion solide comprend de 0,5 à 50 % en poids de nanocellulose sur la base du poids de dérivés de cellulose compris dans la phase continue solide.

15. Procédé de fabrication de l'article hygiénique selon l'une quelconque des revendications 1 à 12, comprenant les étapes consistant à

- préparer un film d'émulsion solide en appliquant une émulsion fluide sur un substrat, et
- sécher l'émulsion fluide jusqu'à ce que 50 à 100 % en poids de l'eau comprise dans l'émulsion fluide se soient évaporés, de manière à obtenir une émulsion solide ; et
- retirer le substrat ;

ou

- préparer une mousse d'émulsion solide en ajoutant un agent moussant à une émulsion fluide ;
- appliquer l'émulsion sur un substrat ;
- laisser l'émulsion fluide mousser ;
- sécher l'émulsion moussée jusqu'à ce que 50 à 100 % de l'eau comprise dans l'émulsion fluide se soient évaporés, de manière à obtenir une émulsion de mousse solide ;
- retirer le substrat ;

ou

- préparer un matériau particulaire d'émulsion solide en appliquant une émulsion fluide sur un substrat ;
- sécher l'émulsion fluide jusqu'à ce qu'au moins 50 à 100 % de l'eau comprise dans l'émulsion fluide se soient évaporés, de manière à obtenir une émulsion solide ;
- désintégrer mécaniquement l'émulsion solide en particules

avant

- d'incorporer le film, ou la mousse, ou le matériau particulaire dans un article hygiénique, dans lequel l'émulsion fluide est constituée par une phase continue comprenant de l'eau, des dérivés de cellulose (CD) et de la nanocellulose et une phase dispersée liquide comprenant une composition contenant de l'huile ; dans lequel l'émulsion solide comprend une matrice de phase continue solide et de phase huileuse dispersée, la phase continue solide comprenant les dérivés de cellulose choisis parmi la carboxyméthylcellulose (CMC), l'hydroxyéthylcellulose (HEC), l'hydroxyéthylcellulose d'éthyle (EHEC), l'hydroxypropylméthylcellulose (HPMC), ou des combinaisons de deux ou plus de ces dérivés de cellulose, et la nanocellulose, la phase huileuse dispersée comprenant la composition contenant de l'huile, et la phase continue solide de l'émulsion solide comprend de 0,5 à 50 % en poids de nanocellulose sur la base du poids de dérivés de cellulose compris dans la phase continue solide.

Fig. 1

Fig. 2

Fig. 3

EHEC/CNC
HPMC/CNC

HEC/CNC
CMC/CNC

● CD ━━CNC ◯Oil ▨Water

Fig. 4

Fig. 5

Fig. 6

Fig. 7

Fig. 8

Fig. 9

(246μm x 246μm)

Fig. 10

(246µm x 246µm)

Fig. 11

Fig. 12

Fig. 13

Fig. 14

Fig. 15

| 2,5% CMC | 2,0% CMC | 1,5% CMC | 1,0% CMC |
| 0%  CNC | 0,5% CNC | 1,0% CNC | 1,5% CNC |

Fig. 16

A dry film Solid Emulsion HPMC+CNC with oil

Thickness 900μm

Fig. 17a

Fig. 17b

Fig. 17c

Fig. 18

Fig. 19a

Fig. 19b

Fig. 19c

Fig. 20

Fig. 21a

Fig. 21b

Fig. 22

Fig. 23a

Fig. 23b

Fig. 24

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2004266302 A
- WO 2013009225 A1
- WO 2010071584 A1

**Non-patent literature cited in the description**

- **HU et al.** Synergetic Stabilization of Emulsions and Emulsion Gels with Water Soluble-Polymers and Cellulose Nanocrystals. *ACS Sustainable Chemistry and Engineering,* 04 May 2015, vol. 3 (5), 1023-1031